# EUROPEAN PATENT SPECIFICATION

(11) **EP 3 849 666 B1**
(45) Date of publication and mention of the grant of the patent: **15.04.2026**
(21) Application number: 19795641.0
(22) Date of filing: 13.09.2019
(51) Int. Cl.: A61P 35/00, C07K 16/22, C07K 16/28, A61K 39/00

(54) **METHODS FOR MODULATING REGULATORY T CELLS AND INHIBITING TUMOR GROWTH**
VERFAHREN ZUR MODULATION VON REGULATORISCHEN T-ZELLEN UND ZUR INHIBIERUNG VON TUMORWACHSTUM
METHODE POUR LA MODULATION DES CELLULES T REGULATRICES ET POUR L'INHIBITION DE LA CROISSANCE TUMORALE

(30) Priority: 14.09.2018 US 201862731351 P
(43) Date of publication of application: 21.07.2021
(73) Proprietor: Université de Lausanne, 1011 Lausanne (CH)
(72) Inventor: HO, Ping-Chih, 1066 Epalinges (CH); WANG, Haiping, 1066 Epalinges (CH)
(74) Representative: HGF
(86) International application number: PCT/IB2019/057745
(87) International publication number: WO 2020/053833

(56) References cited:
- WO-A1-2016/196912
- WO-A1-2017/055411
- WANG H.P. & HO P.C.: "CD36-mediated metabolic adaptation guides regulatory T-cells in tumors", CANCER IMML. RES., vol. 7, no. 2S, A223, 2019, Retrieved from the Internet <URL:https://aacrjournals.org/cancerimmunolres/article/7/2_Supplement/A223/469213/Abstract-A223-CD36-mediated-metabolic-adaptation> [retrieved on 20230608], DOI: 10.1158/2326-6074.CRICIMTEATIAACR18-A223
- AL-KHAMI AMIR A. ET AL: "Exogenous lipid uptake induces metabolic and functional reprogramming of tumor-associated myeloid-derived suppressor cells", ONCOIMMUNOLOGY, vol. 6, no. 10, 30 June 2017 (2017-06-30), pages e1344804, XP093015368, DOI: 10.1080/2162402X.2017.1344804
- WANG HAIPING ET AL: "Metabolic Regulation of Tregs in Cancer: Opportunities for Immunotherapy", TRENDS IN CANCER, vol. 3, no. 8, 1 August 2017 (2017-08-01), pages 583 - 592, XP093015372, ISSN: 2405-8033, DOI: 10.1016/j.trecan.2017.06.005
- DENNIS LINDAU ET AL: "The immunosuppressive tumour network: myeloid-derived suppressor cells, regulatory T cells and natural killer T cells", CANCER RESEARCH, WILEY-BLACKWELL PUBLISHING LTD, GB, vol. 138, no. 2, 16 January 2013 (2013-01-16), pages 105 - 115, XP071276372, ISSN: 0019-2805, DOI: 10.1111/IMM.12036
- GLORIA PASCUAL ET AL: "Targeting metastasis-initiating cells through the fatty acid receptor CD36", NATURE, vol. 541, no. 7635, 7 December 2016 (2016-12-07), pages 41 - 45, XP055584233, DOI: 10.1038/nature20791
- BERRE STEFANO ET AL: "CD36-specific antibodies block release of HIV-1 from infected primary macrophages and its transmission to T cells", JOURNAL OF EXPERIMENTAL MEDICINE, vol. 210, no. 12, 21 October 2013 (2013-10-21), US, pages 2523 - 2538, XP093236699, ISSN: 0022-1007, Retrieved from the Internet <URL:https://rupress.org/jem/article-pdf/210/12/2523/1745329/jem_20130566.pdf> DOI: 10.1084/jem.20130566
- YANG P. ET AL.: "Dietary oleic acid-induced CD36 promotes cervical cancer cell growth and metastasis via up-regulation Src/ERK pathway.", CANCER LETTERS, vol. 438, 11 September 2018 (2018-09-11), pages 76 - 85, XP002796200
- WANG X. ET AL.: "PPAR-delta promotes survival of breast cancer cells in harsh metabolic conditions", ONCOGENESIS, vol. 5, E232, 2016, XP002796201
- HIRSCHHORN-CYMERMAN D. ET AL: "OX40 engagement and chemotherapy combination provides potent antitumor immunity with concomitant regulatory T cell apoptosis.", J. EXP. MED., vol. 206, no. 5, 4 May 2009 (2009-05-04), pages 1103 - 1116, XP002796202
- M. A. CURRAN ET AL: "PD-1 and CTLA-4 combination blockade expands infiltrating T cells and reduces regulatory T and myeloid cells within B16 melanoma tumors", PROCEEDINGS OF THE NATIONAL ACADEMY OF SCIENCES, vol. 107, no. 9, 16 February 2010 (2010-02-16), pages 4275 - 4280, XP055568328, ISSN: 0027-8424, DOI: 10.1073/pnas.0915174107

## Description

### TECHNICAL FIELD OF THE INVENTION

This invention relates to methods for modulating intratumoral regulatory T (Treg) cells and inhibiting tumor growth in a subject and more specifically relates to methods for modulating intratumoral Treg cells and inhibiting tumor growth in a subject by a CD36 inhibitor or a PPARβ inhibitor.

### BACKGROUND OF THE INVENTION

Regulatory T (Treg) cells are a distinct population of T cells which modulate the immune system, maintain tolerance to self-antigens, suppress the aberrant activation of self-reactive T-cells, and abrogate autoimmune disease. Treg cells mediate their regulatory function through a number of mechanisms. First, Treg cells express anti-inflammatory cytokines including IL-10, TGFβ, and IL-35. Another mechanism of regulation is by cell-to-cell contact. Cytotoxic T-lymphocyte antigen-4 (CTLA-4) expressed on Treg cells binds to co-stimulatory B7 molecules on antigen-presenting cells (APC) with about 10-fold higher affinity than CD28, and thus prevent APC from activating naive T cells. Treg cells have also been proposed to prevent differentiation of effector T-cells by consuming cytokines (*e.g.*, IL-2, IL-4, IL-7) required for T-cell activation and polarization (Ward-Hartstonge and Kemp. Clinical & Translational Immunology, 6:9 (2017)).

One mechanism used by cancer cells to evade immune surveillance is the induction of Treg cells which, in turn, suppress the organism's natural immune responses. It is contemplated that, by inactivating Treg cells, the suppression of the immune system could be averted and the immune system would be able to mount a response to destroy primary and metastasized tumors. It has been shown that depleting Treg cells unleashes anti-tumor immunity and interrupts the formation of an immunosuppressive tumor microenvironment (TME). However, systemic loss of Treg cells due to Treg depletion often leads to severe autoimmunity (Wang, H., et al. Trends Cancer 3, 583-592 (2017)).

Tregs are found at high frequencies in both mouse and human cancers (Roychoudhuri, R., Eil, R. L. & Restifo, N. P. The interplay of effector and regulatory T cells in cancer. Current opinion in immunology 33, 101-111 (2015); Delgoffe, G. M. et al. Nature 501, 252-256 (2013); Saito, T. et al. Nat Med 22, 679-684 (2016)), where they represent a major barrier to anti-tumor immunity and cancer immunotherapy (Rech, A. J. et al. Sci Transl Med 4, 134ra162 (2012); Sutmuller, R. P.et al. J Exp Med 194, 823-832 (2001)). While strategies depleting Tregs increase anti-tumor responses, the severe autoimmunity caused by systemic loss of Tregs and the unwanted depletion of effector T cells limit the therapeutic potential of Treg-targeting approaches. In addition, systemic impairment of suppressive functions in Tregs upon treatments targeting immune checkpoints, such as OX40, GITR and CTLA-4, expressing in Tregs also hampers the application of Treg-targeting approaches in cancer treatment (Nishikawa, H. & Sakaguchi, S. International journal of cancer 127, 759-767 (2010); Simpson, T. R. et al. J Exp Med 210, 1695-1710 (2013); Curtin, J. F. et al. PLoS One 3, e1983 (2008)). To date, the search for effective targeting approaches that selectively demolish intratumoral Tregs remains a challenge for cancer immunotherapy.

Al-Khami et al (2017, Oncomimmunology 6(10):e1344804) teaches that CD36 deletion delays tumour growth and alters immunometabolic activity of tumor-infiltrating MDSCs in mice, and that the depletion of CD8+, but not CD4+ T cells eliminated this anti-tumor effect.

Accordingly, there remains a pressing need for a method specifically targeting intratumoral Treg cells to induce anti-tumor immunity without eliciting autoimmunity responses.

### SUMMARY OF THE INVENTION

Any references in the description to methods of treatments refer to the products (e.g., antibodies), pharmaceutical compositions and medicaments of the present invention for use in a method for treatment of the human (or animal) body by therapy (or for diagnosis).

Provided herein are methods to reduce the number of intratumoral Treg cells in a subject and methods to increase the number of intratumoral cytotoxic T-cells in a subject. The methods can be used to inhibit tumor growth in a subject having a cancer.

The invention herein is as described in the claims.

In one aspect, the disclosure provides an CD-36 inhibitor for use in a method of treating cancer by reducing the number of intratumoral CD4+ T regulatory cells, wherein the treatment inhibits tumor growth in a subject having cancer; and wherein the CD-36 inhibitor is an anti-CD36 antibody.

The method of treatment includes administering to the subject a therapeutically effective amount of a CD36 inhibitor alone or in combination with an additional therapeutic agent.

The CD36 inhibitor is an anti-CD36 antibody. The anti- CD36 antibody may be a human antibody, a humanized antibody, a chimeric antibody, or a bispecific antibody.

The additional therapeutic agent may be an immune checkpoint modulator, such as an antibody specific for the immune checkpoint. Examples of immune checkpoints may include CTLA-4, PD-1, PD-L1, PD-L2, killer immunoglobulin receptor (KIR), LAG3, B7-H3, B7-H4, TIM3, A2aR, CD40L, CD27, OX40, 4-IBB, TCR, BTLA, ICOS, CD28, CD80, CD86, ICOS-L,

HVEM, 4-1BBL, OX40L, CD70, CD40, and GALS. In some embodiments, the additional therapeutic agent includes a PD-1 inhibitor. In some embodiments, the additional therapeutic agent includes a CTLA-4 inhibitor. In some embodiments, the additional therapeutic agent includes both a PD-1 inhibitor and a CTLA-4 inhibitor.

The CD36 inhibitor may be administered intratumorally, intravenously, subcutaneously, intraosseously, in sustained release, in controlled release, or in delayed release. The additional therapeutic agent may be administered intratumorally, intravenously, subcutaneously, intraosseously, orally, transdermally, in sustained release, in controlled release, in delayed release, as a suppository, or sublingually.

### BRIEF DESCRIPTION OF THE DRAWINGS

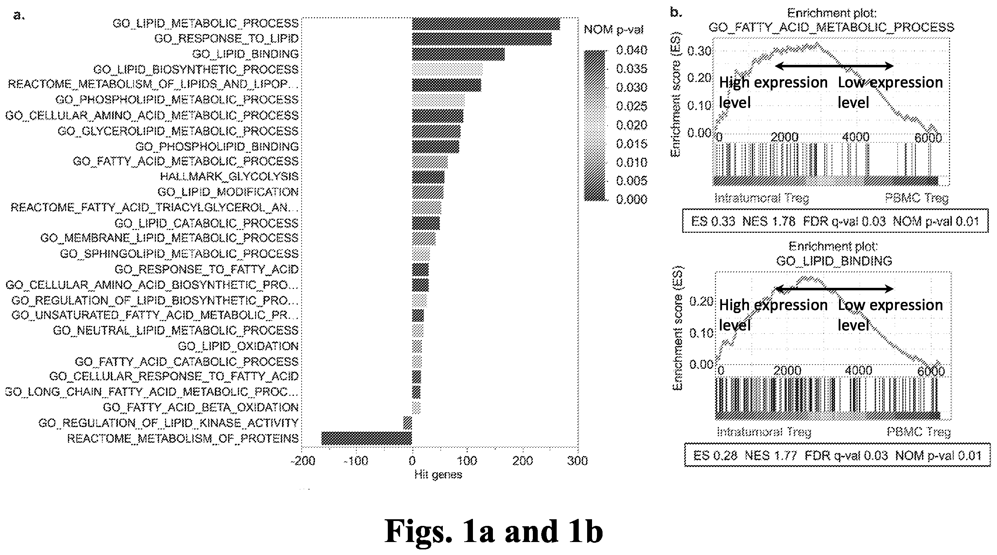

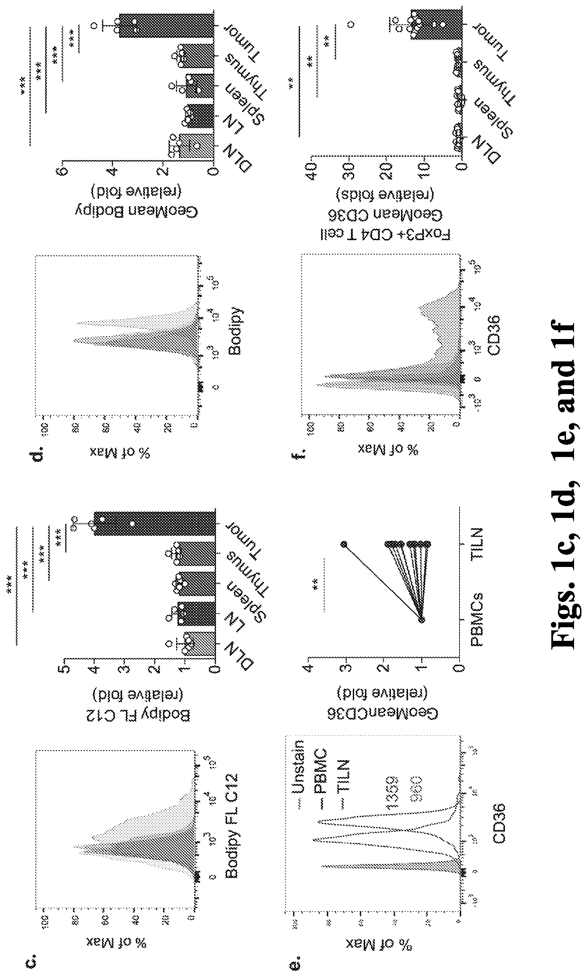

**Figs. 1a, 1b****,** **1c****,** **1d****,** **1e**, and **1f** are a set of diagrams showing that intratumoral Tregs elevated expression of CD36 and genes involved in lipid metabolism. **Fig. 1a** shows pathway enrichment analysis focusing on metabolic machineries for RNA expression in Tregs from breast cancers and PBMC of breast cancer patients. Pathways with significant differential expression between intratumoral and PBMC Tregs (P<0.05) were presented. **Fig. 1b** shows enrichment plots of fatty acid metabolic process (top) and lipid binding (bottom) pathways in intratumoral Treg compared to PBMC Tregs, identified by gene set enrichment analysis (GSEA). Heatmaps show the expression level of each signature gene. Columns indicate individual samples, and rows are each gene. High expression levels and low expression levels are indicated. **Figs. 1e** and **1d** show representative histogram (left) and quantitative results of geometric mean (GeoMean) fluorescent intensity (right) of Bodipy FL C12 (**Fig. 1c**) and Bodipy 493/503 (**Fig. 1d**) in Tregs from indicated tissues of Yumm1.7 melanoma-bearing B6 mice. DLN: draining lymph node (n≥5); LN: non-draining lymph node (n=5); spleen (n≥5); thymus (n=5); tumor (n≥5). **Figs. 1e** and **1f** show representative histogram (left) and quantitative results of geometric mean (GeoMean) fluorescent intensity of CD36 surface staining in Tregs from PBMC and TILs of melanoma patients (n=12) (**Fig. 1e**) and from indicated tissues of Yumm1.7 melanoma-bearing B6 mice (DLN, n=15; spleen, n=15; thymus, n=9; tumor, n=14) (**Fig. 1f**). TILs: tumor-infiltrating lymphocytes. Data are representative results of three independent experiments (**Figs. 1c** and **1d**) or cumulative results from three independent experiments (**Figs. 1e** and **1f**). Each symbol represents one individual. Data are mean± S.D. and were analyzed by two-tailed, unpaired Student's t-test. ***P* < *0.01,* *** *P* < 0.001.

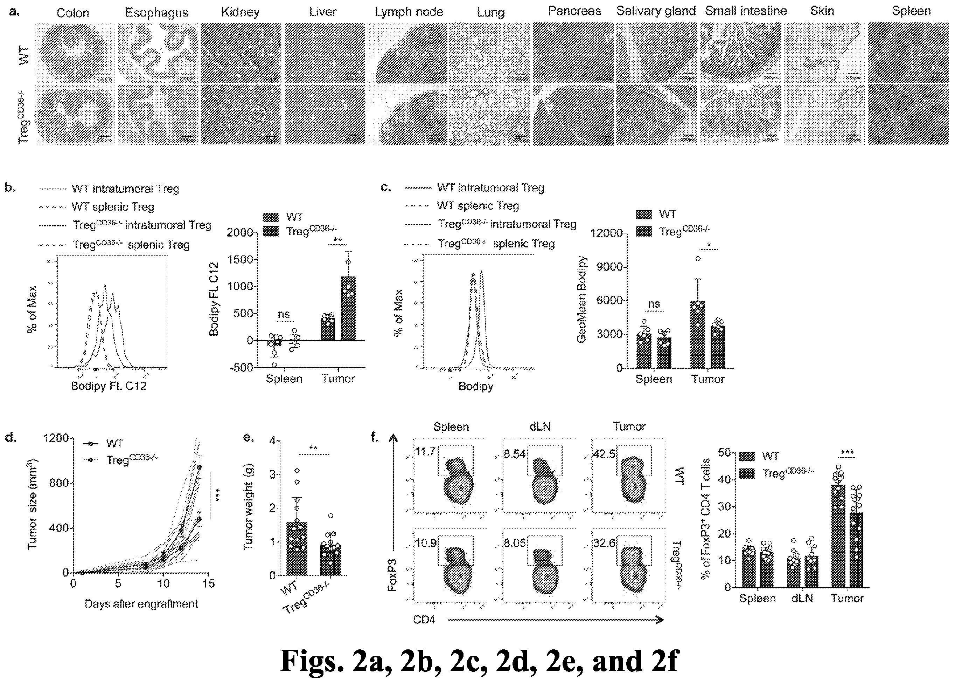

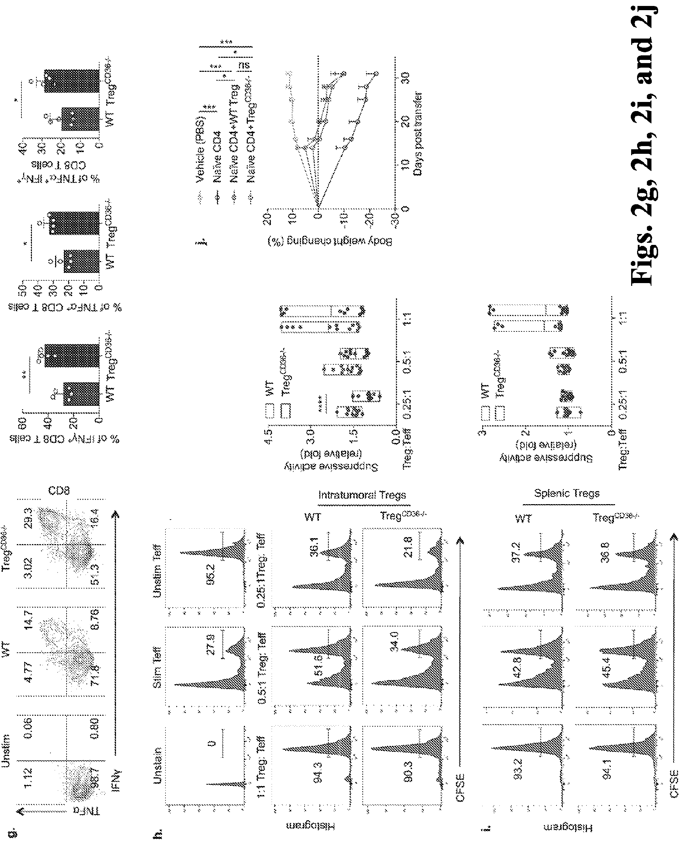

**Figs. 2a, 2b, 2c, 2d, 2e,** **2f****,** **2g****,** **2h****,** **2i**, and **2j** are a set of diagrams showing disruption of CD36 selectively impaired the accumulation and suppressive function of intratumoral Tregs. **Fig. 2a** shows representative images of hematoxylin and eosin (H&E) staining for indicated tissues from WT or Treg^{CD36-/-} mice at the age of 21-23 weeks. Scale bar, 200 µm. **Figs. 2b** and **2c** show representative histogram (left) and quantitative results of geometric mean (GeoMean) fluorescent intensity (right) of Bodipy FL C12 **(****Fig. 2b****)** and Bodipy 493/503 **(****Fig. 2c****)** in splenic and intratumoral Tregs from Yumm1.7 melanoma-bearing WT or Treg^{CD36-/-} mice (n=6 per group). **Figs. 2d** and **2e** show tumor growth **(****Fig. 2d****)** and tumor weight **(Fig. 2e)** of YUMM1.7 melanoma from wild type (WT) or Treg^{CD36-/-} mice (WT, n>9; Treg^{CD36-/-}, n>13). *Foxp3*^{YFP-Cre} mice were used as WT mice. **Fig. 2f** shows representative plots (left) and percentage of FoxP3⁺ Tregs among CD4⁺ T cells in indicated tissues of tumor-bearing WT and Treg^{CD36-/-} mice (spleen, n>12; LN, n>11; tumor, n>13). **Fig. 2g** shows representative plots (left) and percentage of indicated cytokine-producing CD8⁺ T cells among total tumor-infiltrating CD8⁻ T cells from indicated mice (right) (n=5 per group). **Figs. 2h** and **2i** showe*x vivo* suppression of CFSE-labeled WT naive CD8⁺ T cell proliferation by WT and Treg^{CD36-/-} Tregs sorted from tumors (**Fig. 2h**) or spleens **(****Fig. 2i****)** with annotated ratios. **Fig. 2j** shows body weight measurement in Rag1^{-/-} mice receiving naive CD4⁺ T cell alone or in combination with either WT or Treg^{CD36-/-} Tregs (vehicle, n=4; naive CD4, n=7; WT Treg, n=6; Treg^{CD36-/-}, n=6). Data are representative result of at least two independent experiments **(****Figs. 2b, 2c****,** and **2g****)** or cumulative results from three independent experiments **(****Figs. 2d****,** **2e****,** **2f****,** **2h****,** **2i****,** and **2j****).** Each symbol represents one individual. Data are mean± S.D. **(****Figs. 2b, 2c, 2e,** **2f****,** **2g, 2h****,** and **i)** or± S.E.M. **(****Figs. 2d** and **2j****)** and were analyzed by two-tailed, unpaired Student's *t*-test **(****Figs. 2b, 2c, 2d, 2e,** **2f****,** **2g****,** **2h****,** and **2i****)** or one-way ANOVA with Tukey's multiple comparison test in **(****Fig. 2j****).** **P* < *0.05, **P* < *0.01, ***P* < 0.001, **** *P* < 0.0001, ns, no significant difference.

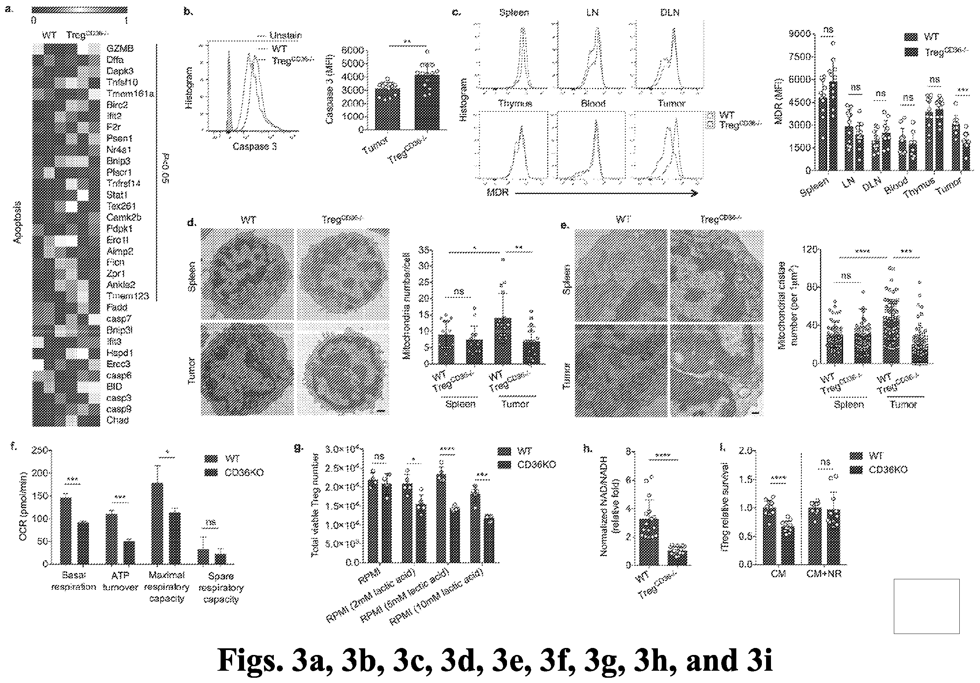

**Figs. 3a, 3b, 3c, 3d, 3e, 3f, 3g, 3h,** and **3i** are a set of diagrams showing CD36 deficiency stimulated apoptosis in intratumoral Tregs. **Fig. 3a** shows expression of genes related to apoptotic pathways in WT and Treg^{CD36-/-} intratumoral Tregs, assessed by RNA-seq (n=3 per group). Differentially expressed genes with P value< 0.05 were indicated. **Fig. 3b** shows representative histograms (left) and quantitative analysis (right) of cleaved caspase-3 levels in intratumoral Tregs from WT (n=13) and Treg^{CD36-/-} tumor-bearing mice (n=14). **Fig. 3c** shows representative histograms (left) and quantitative analysis (right) of MitoTracker Deep Red (MDR) staining in Tregs of spleen, non-draining lymph node (LN), draining lymph node (DLN), blood, thymus, and tumor from tumor-bearing WT and Treg^{CD36-/-} mice (n>8 per group). **Figs. 3d** and **3e** show representative electron microscope images (left) and quantitative plots (right) of mitochondrion number **(****Fig. 3d****)** and crista density **(****Fig. 3e****)** in splenic and intratumoral Tregs from tumor-bearing WT and Treg^{CD36-/-} mice. Scale bars: 500 nm in **(****Fig. 3d****)** and 200 nm in (**Fig. 3e**). **Fig. 3f** shows OCR of indicated iTreg cultured in cancer cell-conditioned medium for 48 hrs (n≥4 per group). **Fig. 3g** shows the viability of either WT or Treg^{CD36-/-} iTreg cultured in cancer cell-conditioned medium as above and then treated with indicated concentration of lactic acids for another 72 hrs (n≥4 per group). **Fig. 3h** shows NAD/NADH ratio of indicated iTreg cultured in cancer cell-conditioned medium for 48 hrs (WT, n=11; Treg^{CD36-/-}, n=13). **Fig. 3i** shows the relative viability of either WT or Treg^{CD36-/-} iTreg treated with cancer cell-conditioned medium with or without NR (400 µM) for 72 hrs (n=11 per group). Results were normalized to the survival cells of control treatment in indicated group. NR: nicotinamide riboside. Data are representative result of three independent experiments **(****Figs. 3f** and **3g****)** or cumulative results of three independent experiments **(****Figs. 3b, 3c, 3h,** and **3i****).** Data are mean ± S.D. and were analyzed by two-tailed, unpaired Student's *t*-test. **P* < *0.05, **P* < *0.01,* ****P* < 0.001, *****P* < 0.0001, ns, no significant difference.

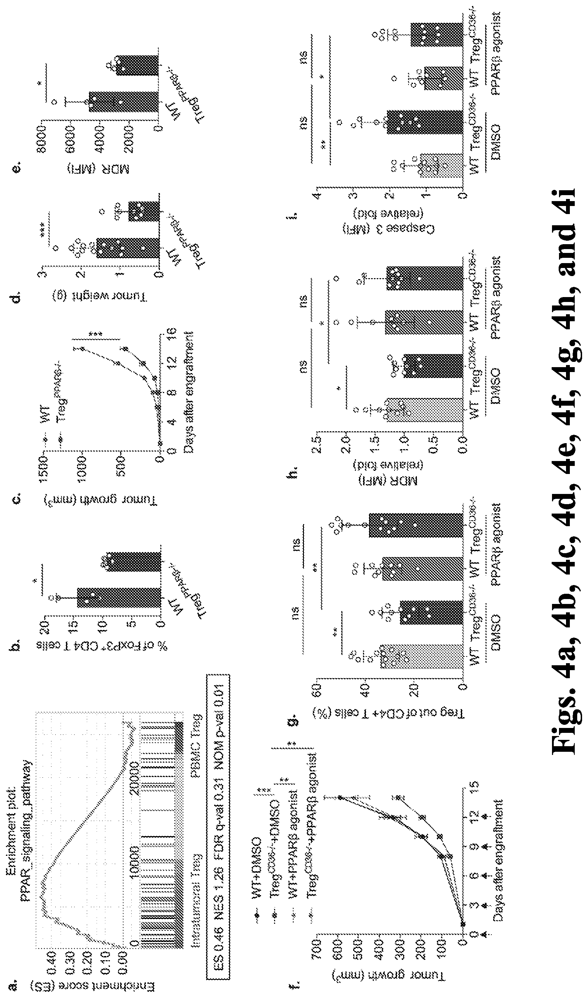

**Figs. 4a, 4b, 4c, 4d, 4e, 4f, 4g, 4h,** and **4i** are a set of diagrams showing that PPARβ signaling is required for metabolic adaptation in intratumoral Tregs. **Fig. 4a** shows enrichment plots of PPAR signaling pathways in intratumoral Treg compared to PBMC Tregs, identified by gene set enrichment analysis (GSEA). Heatmaps show the expression level of each signature gene. **Fig. 4b** shows the percentage of FoxP3⁺ Tregs among CD4⁺ tumor-infiltrating T lymphocytes from tumor-bearing WT and Treg^{PPARβ-/-} mice. **Figs. 4c** and **4d** show tumor growth (**Fig. 4c**) and tumor weight **(****Fig. 4d****)** of YUMM1.7 melanoma from wild type (WT) or Treg^{PPARβ-/-} mice (WT, n=15; Treg^{CD36-/-}, n=10). *Foxp3*^{YFP-Cre} mice were used as WT mice. **Fig. 4e** shows quantitative result of geometric MFI of MDR staining in intratumoral Tregs from WT and Treg^{PPARβ-/-} mice (n=5 per group). **Figs. 4f** and **4g** show that WT and Treg^{CD36-/-} mice were engrafted with YUMM1.7 melanoma cells and then treated with either DMSO or PPARβ agonist as described in methods. Tumor growth **(****Fig. 4f****)** (WT+DMSO, n=6; Treg^{CD36-/-}+DMSO, n=11; WT+PPARβ agonist, n=5; Treg^{CD36-/-}+PPARβ agonist, n=9) and percentage of FoxP3⁺ Tregs among CD4⁺ tumor-infiltrating T lymphocytes **(****Fig. 4g****)** were analyzed (n≥9 per group). **Figs. 4h** and **4i** show quantitative analysis of MitoTracker Deep Red (MDR) staining **(****Fig. 4h****)** and expression of cleaved caspase-3 **(****Fig. 4i****)** in intratumoral Tregs of WT and Treg^{CD36-/-} mice treated with indicated treatments (n≥9 per group). Data are representative result of three independent experiments **(****Figs. 4b** and **4e****)** or cumulative results from at least three independent experiments **(****Figs. 4c, 4d, 4f, 4g, 4h,** and **4i****).** Data are mean ± S.D. (**Figs. 4b, 4d, 4e, 4g, 4h,** and **4i**) or ± S.E.M. (**Figs. 4c** and **4f**) and were analyzed by two-tailed, unpaired Student's *t*-test. **P < 0.05, **P < 0.01,*** P <* 0.001, ns, no significant difference.

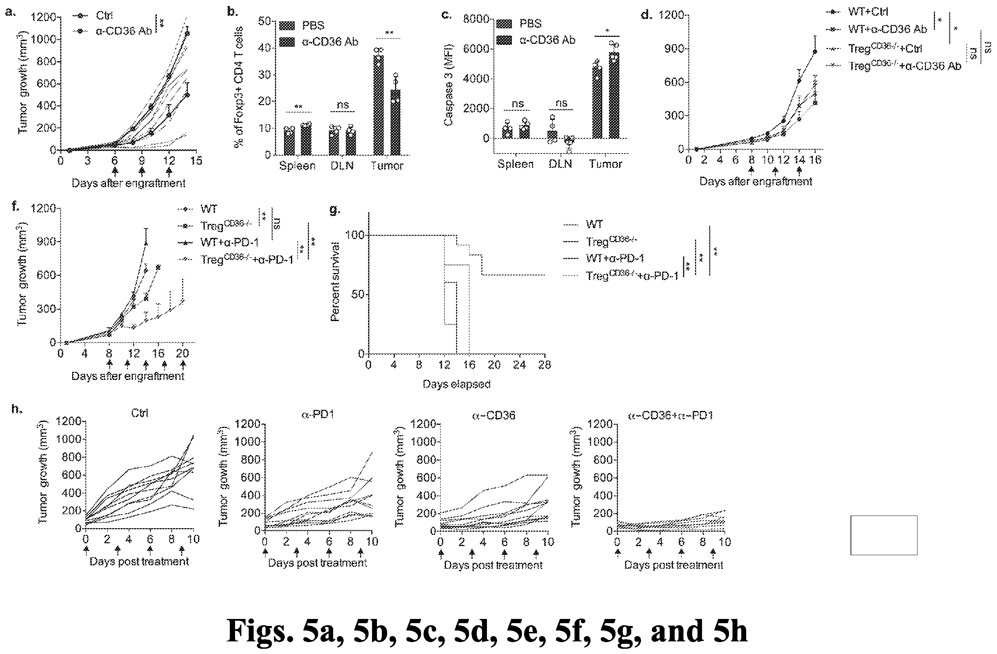

**Figs. 5a, 5b, 5c, 5d, 5e, 5f, 5g,** and **5h** are a set of diagrams showing CD36-targeting impaired intratumoral Tregs and primes tumors to PD-1 blockade. **Figs. 5a, 5b,** and **5c** show tumor growth **(****Fig. 5a****)** (Ctrl, n=4; α-CD36 Ab, n=6), percentage of FoxP3⁺ Tregs among CD4⁺ T cells of indicated tissues **(****Fig. 5b****)** (Ctrl, n=4; α-CD36 Ab, n=4), and expression of cleaved caspase-3 in Tregs isolated from indicated tissues **(****Fig. 5c****)** (Ctrl, n=4; α-CD36 Ab, n=5) of YUMM1.7 melanoma-bearing B6 mice treated with the indicated treatments. **Fig. 5d** shows tumor growth of YUMM1.7 melanoma-bearing WT and Treg^{CD36-/-} mice treated with either control vehicle (Ctrl) or α-CD36 mAb (WT+Ctrl, n=12; WT+a-CD36 Ab, n=10; Treg^{CD36-/-}+Ctrl, n=11; Treg^{CD36-/-}+α-CD36 Ab, n=10). **Fig. 5e** shows tumor growth of YUMM1.7 melanoma-bearing WT and Treg^{CD36}-mice treated with the indicated treatments (WT+Ctrl, n=12; WT+α-CD36 Ab, n=10; Treg^{CD36-/-} +Ctrl, n=11; Treg^{CD36-/-}+α-CD36 Ab, n=10). **Figs. 5f** and **5g** show tumor growth **(****Fig. 5f****)** and Kaplan-Meier survival curves **(****Fig. 5g****)** of YUMM1.7 melanoma-bearing WT and Treg^{CD36-/-} mice treated with the indicated treatments (WT, n=5; Treg^{CD36-/-}, n=4; WT+α-PD1, n=4; Treg^{CD36-1-}+α-PD1, n=5). Differences in survival times were analyzed by long-rank (Mantel-Cox) test. **Fig. 5h** shows tumor growth of inducible Braf/Tten melanoma-bearing mice treated with indicated treatments (Ctrl, n=10; α-PD1, n=11; α-CD36, n=11; α-CD36+αPD-1, n=11). Arrows indicate the date of treatment. Data are representative result of three independent experiments **(****Figs. 5a, 5b,** and **5c****)** or cumulative results from at least two independent experiments **(****Figs. 5d, 5e, 5f, 5g,** and **5h****).** Each symbol represents one individual. Data are mean± S.D. **(****Figs. 5b and 5c****)** or ± S.E.M. **(****Figs. 5a, 5d, 5e, and 5f****)** and were analyzed by two-tailed, unpaired Student's *t-*test. **P < 0.05, **P < 0.01,* ns, no significant difference.

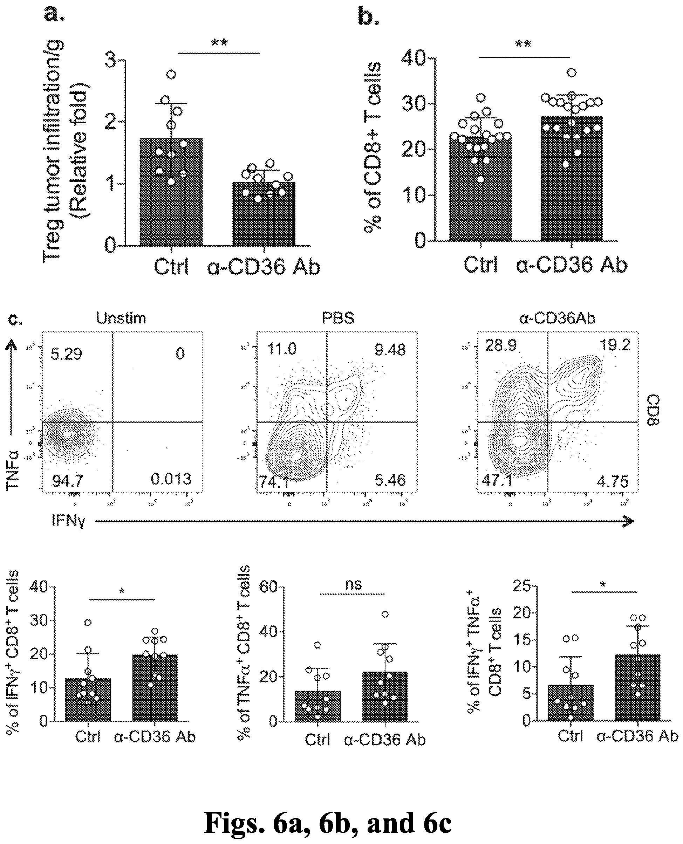

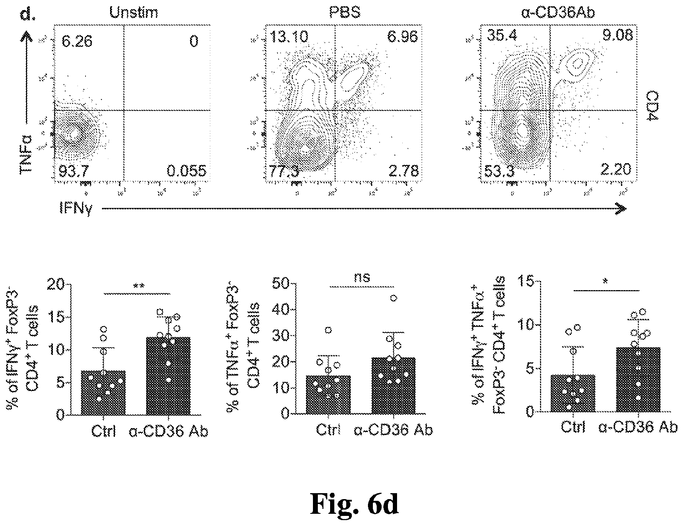

**Figs. 6a, 6b, 6c****,** and **6d** are a set of diagrams showing CD36-targeting unleashed host antitumor immunity. **Figs. 6a, 6b, 6c****,** and **6d** show absolute number of FoxP3⁺ Tregs per gram tumor (n=10 per group) **(****Fig. 6a****),** percentage of CD8⁺ T cells among tumor-infiltrating T cells (n=19 per group) **(****Fig. 6b****)** and representative plots and percentage of indicated cytokine-producing CD8⁺ T cells among total tumor-infiltrating CD8⁺ T cells **(****Fig. 6c****)** and CD4⁺ T cells among total tumor-infiltrating CD4⁺ T cells **(****Fig. 6d****)** (n=10 per group) from YUMM1.7 melanoma-bearing mice treated with indicated treatments.

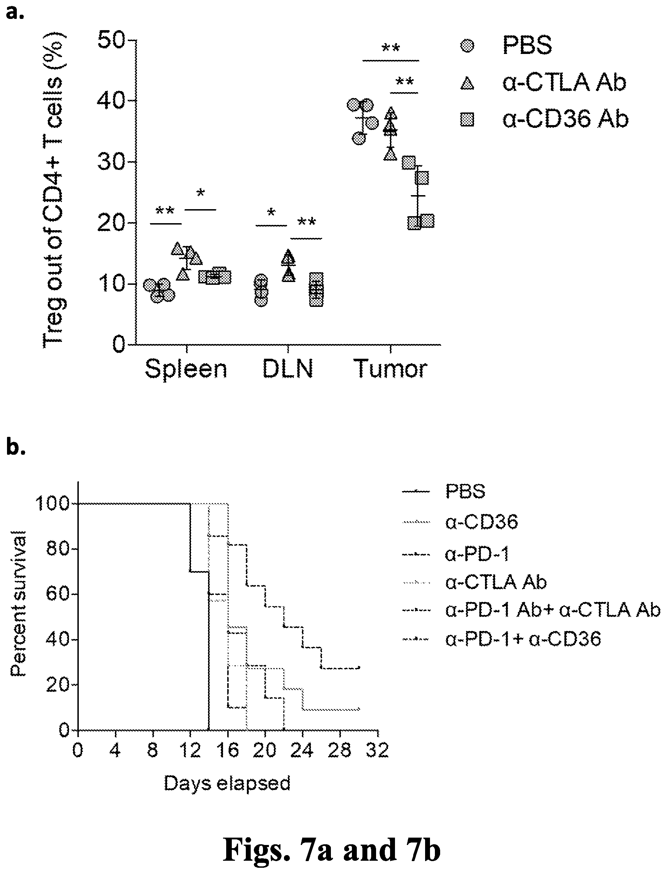

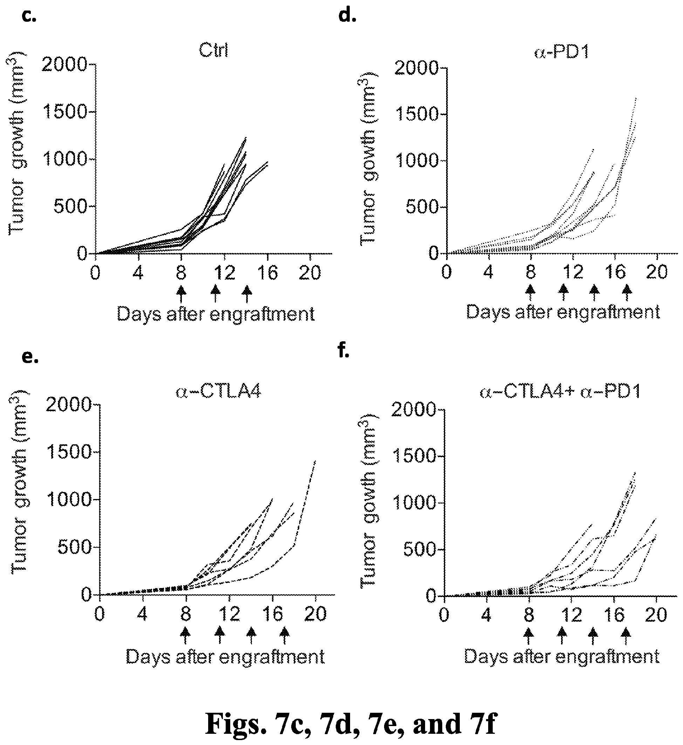

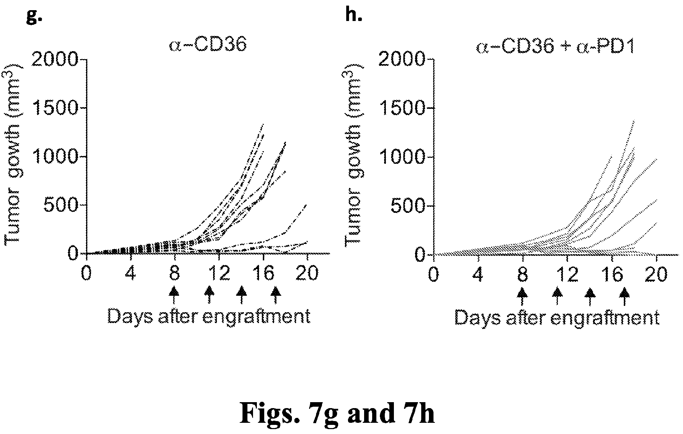

**Figs. 7a, 7b****,** **7c, 7d****,** **7e, 7f****,** **7g,** and **7h** are a set of diagrams showing the synergistic effect of the checkpoint blockade inhibitors. **Fig. 7a** shows percentage of FoxP3+ Tregs among CD4+ T cells of indicated tissues (Ctrl, n=4; α-CTLA4 mAb, n=4; α-CD36 mAb, n=4). Each symbol represents one individual. Data are mean ± S.D. and were analyzed by two-tailed, unpaired Student's t-test. *P < 0.05, **P < 0.01. **Figs. 7b****,** **7c, 7d, 7e****,** **7f****,** **7g,** and **7h** show tumor growth and Kaplan-Meier survival curves of YUMM1.7 melanoma-bearing B6 mice treated with indicated treatments (Ctrl, n=10; α-PD1, n=10; α-CTLA4, n = 7; α-CD36, n = 11; α-CTLA4+αPD-1, n = 7, α-CD36 +αPD-1, n = 11). Arrows indicate the date of treatment. Each symbol represents one individual.

### DETAILED DESCRIPTION OF THE INVENTION

The disclosed methods for modulating intratumoral regulatory T (Treg) cells and inhibiting tumor growth in a subject are in part based on the unexpected discovery that Treg-specific ablation of CD36 reduces the accumulation of intratumoral Treg and suppresses tumor growth. Advantageously, Treg-specific CD36 deficiency does not lead to autoimmunity, and CD36-deficient Treg cells remain their suppressive activity, for example, in restraining CD4 T cell-induced inflammatory bowel disease.

Induction of Treg cells is believed to be an underlying reason for the failure of an immune response to tumor-associated antigens by suppressing tumor-specific T cells, such as CD8+ T cytotoxic cells, from attacking tumor cells. Accordingly, it is contemplated that, by inactivating Treg cells, the suppression of the immune system could be averted and the immune system would be able to mount a response to attack primary and metastasized tumors.

This disclosure demonstrates that inactivation of Treg cells can be induced by CD36 deficiency. CD36 is a surface glycoprotein, also known as fatty acid translocase (FAT). CD36 is involved in inflammatory responses and regulates several functions, such as lipid absorption, lipid storage, and lipid utilization (Glatz and Luiken, JLR, 2018). Tumor-infiltrating T cells have abnormally high fatty acid uptake, high intracellular lipid content, and high expression level of CD36 (Yin et al., J Immunol, 2016; Cui and Kaech, Cancer Immunol Res, 2016). CD36 expression supports the survival of intratumoral Treg cells by fine-tuning their mitochondrial fitness via PPAR signaling. Thus, the high expression level of CD36 in intratumoral Treg cells orchestrates metabolic adaptation of Treg cells in tumors by intervening metabolic regulations and further promotes tumor growth by suppressing the anti-tumor immune responses. Thus, targeting CD36 might represent an attractive therapeutic approach for modulating intratumoral Treg cells and inhibiting tumor growth.

Also disclosed herein, but not claimed, is the targeting of PPAR signalling, which may equally represent an attractive therapeutic approach for the same purpose

The technical disclosure set out below may in some respects go beyond the scope of the claims. Elements of the disclosure which do not fall within the scope of the claims are provided for information.

### L METHODS FOR REDUCING THE NUMBER OF INTRATUMORAL TREG CELLS AND INCREASING THE NUMBER OF INTRATUMORAL CYTOTOXIC T CELLS

Described herein is a CD-36 inhibitor for use in a method of treating cancer by
reducing the number of intratumoral Treg cells in a subject. The method includes administering to the subject an effective amount of a CD36 inhibitor.

Also described is a method of increasing the number of intratumoral cytotoxic T-cells in a subject. The method may include administering to the subject an effective amount of a CD36 inhibitor.

As used herein, a "subject" refers to a human or non-human mammal. Non-human mammals include, for example, livestock and pets, such as ovine, bovine, porcine, canine, feline and murine mammals. In certain embodiments, the subject is human. A "tissue-specific" promoter is a nucleotide sequence that, when operably linked with a polynucleotide encodes or specified by a gene, causes the gene product to be produced in a cell substantially only if the cell is a cell of the tissue type corresponding to the promoter.

As used here, "intratumoral" as it refers to a T cell located within tumor cell islets (*i.e.,* juxtaposed to clearly malignant epithelial cells), while peritumoral T cells are located in the stroma that surrounds and infiltrates a tumor. Thus, a T cell may be located within the tumor, but by virtue of being intimately associated with stromal rather than actual malignant cells, it may not be viewed as an intratumoral T cell. Any method known in the art for detecting a T cell that preserves the tumor architecture may be used to ascertain if it is intratumoral.

As used herein, the term "regulatory T cell" or "Treg cells" refers to a CD4+CD25+ FoxP3+ T cell with suppressive properties. As used herein, the term "helper T cell" refers to a CD4 T cell. Helper T cells (*e.g.,* Th1 and Th2) recognize antigen bound to MHC Class II molecules and produce different cytokines. As used herein, the term "cytotoxic T cell" as used herein refers to a CD8+ T cell. Cytotoxic T cells recognize antigen bound to MHC Class I molecules.

Treg cells may include T cells expressing CD4, CD25, and FOXP3, *e.g.,* CD4+, CD4+CD25+, CD4+ Foxp3+ regulatory T cells. Cytotoxic T-cells, also known as killer T cells, may include CD8+ T cells. For example, the anti-CD36 antibody may be a human antibody, a humanized antibody, a chimeric antibody, or a bispecific antibody.

In some embodiments, reducing the number of intratumoral Treg cells in a subject may include administering to the subject a CD36 inhibitor in conjunction with an additional therapeutic agent, such as an anti-cancer agent.

The additional therapeutic agent may be an immune checkpoint modulator, such as an antibody specific for the immune checkpoint. Examples of immune checkpoints may include, without limitation, CTLA-4, PD-1, PD-L1, PD-L2, killer immunoglobulin receptor (KIR), LAG3, B7-H3, B7-H4, TIM3, A2aR, CD40L, CD27, OX40, 4-IBB, TCR, BTLA, ICOS, CD28, CD80, CD86, ICOS-L, HVEM, 4-1BBL, OX40L, CD70, CD40, and GALS. Non-limiting examples of immune checkpoint modulators include ipilimumab, tremelimumab pembrolizumab, nivolumab, pidilizumab, MPDL3280A, MEDI4736, BMS-936559, MSB0010718C, and AMP-224.

The CD36 inhibitor, may be administered intratumorally, intravenously, subcutaneously, intraosseously, in sustained release, in controlled release, or in delayed release. The additional therapeutic agent may be administered orally, transdermally, as a suppository, or sublingually.

The CD36 inhibitor, or the additional therapeutic agent, may be prepared as a pharmaceutical composition. Injectable formulations may be prepared, packaged, or sold in unit dosage form, such as in ampules or in multi-dose containers containing a preservative. Formulations for parenteral administration include, but are not limited to, suspensions, solutions, emulsions in oily or aqueous vehicles, pastes, and implantable sustained-release or biodegradable formulations. Such formulations may further include one or more additional ingredients, such as suspending, stabilizing, or dispersing agents. In one example of a formulation for parenteral administration, the active ingredient is provided in dry (i.e., powder or granular) form for reconstitution with a suitable vehicle (e.g., sterile pyrogen-free water) prior to parenteral administration of the reconstitued compound.

### II. METHODS FOR INHIBITING TUMOR GROWTH

This disclosure also provides a method of inhibiting tumor growth in a subject having a cancer. The method may include administering to the subject a therapeutically effective amount of a CD36 inhibitor alone or in combination with an additional therapeutic agent *(e.g.,* an anti-cancer agent). Optionally,the method may additionally include administering to the subject a therapeutically effective amount of a PPARβ inhibitor alone or in combination an additional therapeutic agent (*e.g.,* an anti-cancer agent).

The CD36 inhibitor may include, without limitation, an antibody, monoclonal antibody, or humanized monoclonal antibody.

Similarly, the PPARβ inhibitor may include, without limitation, a nucleic acid molecule (*e.g.,* enzymatic nucleic acid molecule, antisense nucleic acid molecule, triplex oligonucleotide, dsRNA, ssRNA, RNAi, siRNA, aptamer, 2,5-A chimera), lipid, steroid, peptide, protein, allozyme, antibody, monoclonal antibody, humanized monoclonal antibody, and small molecule (*e.g.,* antiviral compounds). Non-limiting examples of the small molecule PPARβ inhibitor may include FH535, GSK0660, GSK3787, PT-S58, PT-S77, and ST-247.

The additional therapeutic agent may be an immune checkpoint modulator, such as an antibody specific for the immune checkpoint. Examples of immune checkpoints may include, without limitation, CTLA-4, PD-1, PD-L1, PD-L2, killer immunoglobulin receptor (KIR), LAG3, B7-H3, B7-H4, TIM3, A2aR, CD40L, CD27, OX40, 4-IBB, TCR, BTLA, ICOS, CD28, CD80, CD86, ICOS-L, HVEM, 4-1BBL, OX40L, CD70, CD40, and GALS. Non-limiting examples of immune checkpoint modulators include ipilimumab, tremelimumab pembrolizumab, nivolumab, pidilizumab, MPDL3280A, MEDI4736, BMS-936559, MSB0010718C, and AMP-224.

In some embodiments, the additional therapeutic agent may include one or more antitumor/anticancer agents, including chemotherapeutic agents and immunotherapeutic agents.

A "chemotherapeutic agent" is a chemical compound useful in the treatment of cancer. Examples of chemotherapeutic agents include alkylating agents such as thiotepa and cyclophosphamide (CYTOXANTM); alkyl sulfonates such as busulfan, improsulfan and piposulfan; aziridines such as benzodopa, carboquone, methyldopa, and uredopa; ethylenimines and methylamelamines including altretamine, triethylenemelamine, trietylenephosphoramide, triethylenethiophosphaoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as the enediyne antibiotics (*e.g.* calicheamicin, see, *e.g.,* Agnew Chem. Intl. Ed. Engl. 33:183-186 (1994); dynemicin, including dynemicin A; an esperamicin; as well as neocarzinostatin chromophore and related chromoprotein enediyne antibiotic chromomophores), aclacinomysins, actinomycin, authramycin, azaserine, bleomycins, cactinomycin, carabicin, caminomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; podophyllinic acid; 2-ethylhydrazide; procarbazine; PSK^{®}., razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL^{®}, Bristol-Myers Squibb Oncology, Princeton, N.J.) and doxetaxel (TAXOTERE^{®}, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylornithine (DMFO); retinoic acid; capecitabine; and pharmaceutically acceptable salts, acids or derivatives of any of the above. Also included in this definition are anti-hormonal agents that act to regulate or inhibit hormone action on tumors such as anti-estrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

An "immunotherapeutic agent" is a biological agent useful in the treatment of cancer. Examples of immunotherapeutic agents include atezolizumab, avelumab, blinatumomab, daratumumab, cemiplimab, durvalumab, elotuzumab, laherparepvec, ipilimumab, nivolumab, obinutuzumab, ofatumumab, pembrolizumab, and talimogene.

The method may include administering to a subject a composition containing a CD36 inhibitor, optionally in combination with a PPARβ inhibitor, an immune checkpoint modulator or any combination thereof. The additional therapeutic agent, the CD36 inhibitor, and/or the PPARβ inhibitor may be administered concurrently or sequentially. The CD36 inhibitor, the PPARβ inhibitor or the immune checkpoint modulator may be administered intratumorally, intravenously, subcutaneously, intraosseously, in sustained release, in controlled release, or in delayed release. The PPARβ inhibitor and/or additional therapeutic agent may be administered orally, transdermally, as a suppository, or sublingually.

Cancers may include, but are not limited to, cardiac cancers, including, for example, sarcoma, e.g., angiosarcoma, fibrosarcoma, rhabdomyosarcoma, and liposarcoma; myxoma; rhabdomyoma; fibroma; lipoma and teratoma; lung cancers, including, for example, bronchogenic carcinoma, e.g., squamous cell, undifferentiated small cell, undifferentiated large cell, and adenocarcinoma; alveolar and bronchiolar carcinoma; bronchial adenoma; sarcoma; lymphoma; chondromatous hamartoma; and mesothelioma; gastrointestinal cancer, including, for example, cancers of the esophagus, *e.g.,* squamous cell carcinoma, adenocarcinoma, leiomyosarcoma, and lymphoma; cancers of the stomach, *e.g.,* carcinoma, lymphoma, and leiomyosarcoma; cancers of the pancreas, *e.g.,* ductal adenocarcinoma, insulinoma, glucagonoma, gastrinoma, carcinoid tumors, and vipoma; cancers of the small bowel, *e.g.,* adenocarcinoma, lymphoma, carcinoid tumors, Kaposi's sarcoma, leiomyoma, hemangioma, lipoma, neurofibroma, and fibroma; cancers of the large bowel, *e.g.,* adenocarcinoma, tubular adenoma, villous adenoma, hamartoma, and leiomyoma; genitourinary tract cancers, including, for example, cancers of the kidney, *e.g.,* adenocarcinoma, Wilm's tumor (nephroblastoma), lymphoma, and leukemia; cancers of the bladder and urethra, *e.g.,* squamous cell carcinoma, transitional cell carcinoma, and adenocarcinoma; cancers of the prostate, *e.g.,* adenocarcinoma, and sarcoma; cancer of the testis, *e.g.,* seminoma, teratoma, embryonal carcinoma, teratocarcinoma, choriocarcinoma, sarcoma, interstitial cell carcinoma, fibroma, fibroadenoma, adenomatoid tumors, and lipoma; liver cancers including, for example, hepatoma, *e.g.,* hepatocellular carcinoma; cholangiocarcinoma; hepatoblastoma; angiosarcoma; hepatocellular adenoma; and hemangioma; bone cancer including, for example, osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochondroma (osteocartilaginous exostoses), benign chondroma, chondroblastoma, chondromyxofibroma, osteoid osteoma and giant cell tumors; nervous system cancers including, for example, cancers of the skull, *e.g.,* osteoma, hemangioma, granuloma, xanthoma, and osteitis deformans; cancers of the meninges, *e.g.,* meningioma, hemangiosarcoma, and gliomatosis; cancers of the brain, *e.g.,* astrocytoma, medulloblastoma, glioma, ependymoma, germinoma (pinealoma), glioblastoma multiform, oligodendroglioma, schwannoma, retinoblastoma, and congenital tumors; and cancers of the spinal cord, *e.g.,* neurofibroma, meningioma, glioma, and sarcoma; gynecological cancers including, for example, cancers of the uterus, *e.g.,* endometrial carcinoma; cancers of the cervix, *e.g.,* cervical carcinoma, and pre-tumor cervical dysplasia; cancers of the ovaries, *e.g.,* ovarian carcinoma, including serous cystadenocarcinoma, mucinous cystadenocarcinoma, unclassified carcinoma, granulosa-thecal cell tumors, Sertoli-Leydig cell tumors, dysgerminoma, and malignant teratoma; cancers of the vulva, *e.g.,* squamous cell carcinoma, intraepithelial carcinoma, adenocarcinoma, fibrosarcoma, and melanoma; cancers of the vagina, *e.g.,* clear cell carcinoma, squamous cell carcinoma, botryoid sarcoma, and embryonal rhabdomyosarcoma; and cancers of the fallopian tubes, *e.g.,* carcinoma; hematologic cancers including, for example, cancers of the blood, *e.g.,* acute myeloid leukemia, chronic myeloid leukemia, acute lymphoblastic leukemia, chronic lymphocytic leukemia, myeloproliferative diseases, multiple myeloma, and myelodysplastic syndrome, Hodgkin's lymphoma, non-Hodgkin's lymphoma (malignant lymphoma) and Waldenström's macroglobulinemia; skin cancers including, for example, malignant melanoma, basal cell carcinoma, squamous cell carcinoma, Kaposi's sarcoma, moles dysplastic nevi, lipoma, angioma, dermatofibroma, keloids, and psoriasis; breast cancers including, for example, ductal carcinoma, lobular carcinoma, inflammatory breast cancer, medullary carcinoma, mucinous (colloid) carcinoma, Paget's disease of the breast, tubular carcinoma, phyllodes tumor, metaplastic carcinoma, sarcoma, microcapillary carcinoma and adenoid cystic carcinoma; and adrenal gland cancers including, for example, neuroblastoma.

Cancers may be solid tumors that may or may not be metastatic. Cancers may also occur, as in leukemia, as a diffuse tissue.

CD36 inhibitors, PPARβ inhibitors, or additional therapeutic agents may be administered to the patient either prior to or after the manifestation of symptoms associated with the disease or condition. Further, several divided dosages, as well as staggered dosages may be administered daily or sequentially, or the dose may be continuously infused or may be a bolus injection. Further, the dosages of the therapeutic formulations may be proportionally increased or decreased as indicated by the exigencies of the therapeutic or prophylactic situation.

Administration to a subject may be carried out using known procedures, at dosages and for periods of time effective to treat a disease or condition in the patient. An effective amount of the therapeutic compound necessary to achieve a therapeutic effect may vary according to factors such as the activity of the particular compound employed; the time of administration; the rate of excretion of the compound; the duration of the treatment; other drugs, compounds or materials used in combination with the compound; the state of the disease or disorder, age, sex, weight, condition, general health and prior medical history of the patient being treated, and like factors well-known in the medical arts. Dosage regimens may be adjusted to provide the optimum therapeutic response. For example, several divided doses may be administered daily or the dose may be proportionally reduced as indicated by the exigencies of the therapeutic situation. A non-limiting example of an effective dose range for a therapeutic compound of the invention is from about 0.01 and 50 mg/kg of body weight/per day. One of ordinary skill in the art would be able to study the relevant factors and make the determination regarding the effective amount of the therapeutic compound without undue experimentation.

Administration can be carried out as frequently as several times daily, or it may be administered less frequently, such as once a day, once a week, once every two weeks, once a month, or even less frequently, such as once every several months or even once a year or less. It is understood that the amount of compound dosed per day may be administered, in non-limiting examples, every day, every other day, every 2 days, every 3 days, every 4 days, or every 5 days. For example, with every other day administration, a 5 mg per day dose may be initiated on Monday with a first subsequent 5 mg per day dose administered on Wednesday, a second subsequent 5 mg per day dose administered on Friday, and so on. The frequency of the dose will be readily apparent to the skilled artisan and will depend upon any number of factors, such as, but not limited to, the type and severity of the disease being treated, the type and age of the animal, etc.

Routes of administration may include inhalational, oral, nasal, rectal, parenteral, sublingual, transdermal, transmucosal (*e.g.,* sublingual, lingual, (trans)buccal, (trans)urethral, vaginal (*e.g.,* trans- and perivaginally), (intra)nasal, and (trans)rectal), intravesical, intrapulmonary, intraduodenal, intragastrical, intrathecal, subcutaneous, intramuscular, intradermal, intracranial, intra-arterial, intravenous, intrabronchial, inhalation, and topical administration.

As used herein, "parenteral administration" includes any route of administration characterized by physical breaching of a tissue of a subject and administration of the pharmaceutical composition through the breach in the tissue. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration can also include, but is not limited to, intracranial, subcutaneous, intravenous, intraperitoneal, intramuscular, intrasternal injection, and kidney dialytic infusion techniques.

CD36 inhibitors, PPARβ inhibitors, or additional therapeutic agents can be provided in various suitable compositions and dosage forms, including, for example, tablets, capsules, caplets, pills, gel caps, troches, dispersions, suspensions, solutions, syrups, granules, beads, transdermal patches, gels, powders, pellets, magmas, lozenges, creams, pastes, plasters, lotions, discs, suppositories, liquid sprays for nasal or oral administration, dry powder or aerosolized formulations for inhalation, compositions and formulations for intravesical administration and the like. It should be understood that the formulations and compositions that would be useful in the present invention are not limited to the particular formulations and compositions that are described herein. For oral application, particularly suitable are tablets, dragees, liquids, drops, suppositories, or capsules, caplets and gelcaps. Other formulations suitable for oral administration include, but are not limited to, a powdered or granular formulation, an aqueous or oily suspension, an aqueous or oily solution, a paste, a gel, toothpaste, a mouthwash, a coating, an oral rinse, or an emulsion.

### III. DEFINITIONS

To aid in understanding the detailed description of the compositions and methods according to the disclosure, a few express definitions are provided to facilitate an unambiguous disclosure of the various aspects of the disclosure. Unless otherwise defined, all technical and scientific terms used herein have the same meaning as commonly understood by one of ordinary skill in the art to which this disclosure belongs.

As used herein, "PPARβ" and "PPARδ" are used interchangeably in the disclosure and refer to the same PPAR receptor.

As used herein, a "subject" refers to a mammal, including a human. Non-human animals subject to diagnosis or treatment may include, for example, primates, cattle, goats, sheep, horses, dogs, cats, mice, rats, and the like.

As used herein, the term "treatment" refers to an intervention performed with the intention of preventing the development or altering the pathology or symptoms of a disorder. Accordingly, "treatment" refers to both therapeutic treatment and prophylactic or preventative measures. Those in need of treatment include those already with the disorder as well as those in which the disorder is to be prevented. In tumor (*e.g.,* cancer) treatment, a therapeutic agent may directly decrease the pathology of tumor cells, or render the tumor cells more susceptible to treatment by other therapeutic agents, *e.g.,* radiation and/or chemotherapy.

Thus, "treating" may include suppressing, inhibiting, preventing, treating, or a combination thereof. Treating refers inter alia to increasing time to sustained progression, expediting remission, inducing remission, augmenting remission, speeding recovery, increasing efficacy of or decreasing resistance to alternative therapeutics, or a combination thereof. "Suppressing" or "inhibiting" refers inter alia to delaying the onset of symptoms, preventing relapse to a disease, decreasing the number or frequency of relapse episodes, increasing latency between symptomatic episodes, reducing the severity of symptoms, reducing the severity of an acute episode, reducing the number of symptoms, reducing the incidence of disease-related symptoms, reducing the latency of symptoms, ameliorating symptoms, reducing secondary symptoms, reducing secondary infections, prolonging patient survival, or a combination thereof.

As used herein, the term "modulate" or "modulating" is meant that any of the mentioned activities are, e.g., increased, enhanced, increased, augmented, agonized (acts as an agonist), promoted, decreased, reduced, suppressed blocked, or antagonized (acts as an antagonist). Modulation can increase activity more than 1-fold, 2-fold, 3-fold, 5-fold, 10-fold, 100-fold, etc., over baseline values. Modulation can also decrease its activity below baseline values.

The terms "prevent," "preventing," "prevention," "prophylactic treatment" and the like refer to reducing the probability of developing a disorder or condition in a subject, who does not have, but is at risk of or susceptible to developing a disorder or condition.

The term "disease" as used herein is intended to be generally synonymous, and is used interchangeably with, the terms "disorder" and "condition" (as in medical condition), in that all reflect an abnormal condition of the human or animal body or of one of its parts that impairs normal functioning, is typically manifested by distinguishing signs and symptoms, and causes the human or animal to have a reduced duration or quality of life.

The terms "decrease," "reduced," "reduction," "decrease," or "inhibit" are all used herein generally to mean a decrease by a statistically significant amount. However, for avoidance of doubt, "reduced", "reduction" or "decrease" or "inhibit" means a decrease by at least 10% as compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (*e.g.* absent level as compared to a reference sample), or any decrease between 10-100% as compared to a reference level.

The terms "increased", "increase" or "enhance" or "activate" are all used herein to generally mean an increase by a statically significant amount; for the avoidance of any doubt, the terms "increased", "increase" or "enhance" or "activate" means an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level.

The term "effective amount," "effective dose," or "effective dosage" is defined as an amount sufficient to achieve or at least partially achieve a desired effect. A "therapeutically effective amount" or "therapeutically effective dosage" of a drug or therapeutic agent is any amount of the drug that, when used alone or in combination with another therapeutic agent, promotes disease regression evidenced by a decrease in severity of disease symptoms, an increase in frequency and duration of disease symptom-free periods, or a prevention of impairment or disability due to the disease affliction. A "prophylactically effective amount" or a "prophylactically effective dosage" of a drug is an amount of the drug that, when administered alone or in combination with another therapeutic agent to a subject at risk of developing a disease or of suffering a recurrence of disease, inhibits the development or recurrence of the disease. The ability of a therapeutic or prophylactic agent to promote disease regression or inhibit the development or recurrence of the disease can be evaluated using a variety of methods known to the skilled practitioner, such as in human subjects during clinical trials, in animal model systems predictive of efficacy in humans, or by assaying the activity of the agent in *in vitro* assays.

Doses are often expressed in relation to bodyweight. Thus, a dose which is expressed as [g, mg, or other unit]/kg (or g, mg etc.) usually refers to [g, mg, or other unit] "per kg (or g, mg etc.) bodyweight", even if the term "bodyweight" is not explicitly mentioned.

The term "agent" is used herein to denote a chemical compound, a mixture of chemical compounds, a biological macromolecule (such as a nucleic acid, an antibody, a protein or portion thereof, *e.g.,* a peptide), or an extract made from biological materials such as bacteria, plants, fungi, or animal (particularly mammalian) cells or tissues. The activity of such agents may render it suitable as a "therapeutic agent," which is a biologically, physiologically, or pharmacologically active substance (or substances) that acts locally or systemically in a subject.

The terms "therapeutic agent," "therapeutic capable agent," or "treatment agent" are used interchangeably and refer to a molecule or compound that confers some beneficial effect upon administration to a subject. The beneficial effect includes enablement of diagnostic determinations; amelioration of a disease, symptom, disorder, or pathological condition; reducing or preventing the onset of a disease, symptom, disorder or condition; and generally counteracting a disease, symptom, disorder or pathological condition.

As used herein, a "therapeutically effective amount" or an "effective amount" refers to a nontoxic but sufficient amount of an agent to provide the desired biological result. That result can be reduction and/or alleviation of the signs, symptoms, or causes of a disease or disorder, or any other desired alteration of a biological system. An appropriate therapeutic amount in any individual case may be determined by one of ordinary skill in the art using routine experimentation.

"Combination" therapy, as used herein, unless otherwise clear from the context, is meant to encompass administration of two or more therapeutic agents in a coordinated fashion, and includes, but is not limited to, concurrent dosing. Specifically, combination therapy encompasses both co-administration (*e.g.,* administration of a co-formulation or simultaneous administration of separate therapeutic compositions) and serial or sequential administration, provided that administration of one therapeutic agent is conditioned in some way on administration of another therapeutic agent. For example, one therapeutic agent may be administered only after a different therapeutic agent has been administered and allowed to act for a prescribed period of time. See, *e.g.,* Kohrt et al. (2011) Blood 117:2423.

As used herein, the term "depletion" refers to reducing or eliminating the function of a given type of cell, rendering the cell ineffective, partially or completely eliminating the proliferation of the cell, and/or killing the cell.

As used herein, the term "antibody" (Ab) includes monoclonal antibodies, polyclonal antibodies, multispecific antibodies (for example, bispecific antibodies and polyreactive antibodies), and antibody fragments. Thus, the term "antibody" as used in any context within this specification is meant to include, but not be limited to, any specific binding member, immunoglobulin class and/or isotype (*e.g.,* IgG1, IgG2, IgG3, IgG4, IgM, IgA, IgD, IgE and IgM); and biologically relevant fragment or specific binding member thereof, including but not limited to Fab, F(ab')2, Fv, and scFv (single chain or related entity). It is understood in the art that an antibody is a glycoprotein having at least two heavy (H) chains and two light (L) chains interconnected by disulfide bonds, or an antigen-binding portion thereof. A heavy chain is comprised of a heavy chain variable region (VH) and a heavy chain constant region (CH1, CH2, and CH3). A light chain is comprised of a light chain variable region (VL) and a light chain constant region (CL). The variable regions of both the heavy and light chains comprise framework regions (FWR) and complementarity determining regions (CDR). The four FWR regions are relatively conserved while CDR regions (CDR1, CDR2, and CDR3) represent hypervariable regions and are arranged from NH2 terminus to the COOH terminus as follows: FWR1, CDR1, FWR2, CDR2, FWR3, CDR3, and FWR4. The variable regions of the heavy and light chains contain a binding domain that interacts with an antigen while, depending on the isotype, the constant region(s) may mediate the binding of the immunoglobulin to host tissues or factors. Also included in the definition of "antibody" as used herein are chimeric antibodies, humanized antibodies, and recombinant antibodies, human antibodies generated from a transgenic non-human animal, as well as antibodies selected from libraries using enrichment technologies available to the artisan.

As used herein, the term *"in vitro"* refers to events that occur in an artificial environment, *e.g.,* in a test tube or reaction vessel, in cell culture, etc., rather than within a multi-cellular organism.

As used herein, the term *"in vivo"* refers to events that occur within a multi-cellular organism such as a non-human animal.

It is noted here that, as used in this specification and the appended claims, the singular forms "a," "an," and "the" include plural reference unless the context clearly dictates otherwise.

The terms "including," "comprising," "containing," or "having" and variations thereof are meant to encompass the items listed thereafter and equivalents thereof as well as additional subject matter unless otherwise noted.

The phrases "in one embodiment," "in various embodiments," "in some embodiments," and the like are used repeatedly. Such phrases do not necessarily refer to the same embodiment, but they may unless the context dictates otherwise.

The terms "and/or" or "/" means any one of the items, any combination of the items, or all of the items with which this term is associated.

The word "substantially" does not exclude "completely," *e.g.,* a composition which is "substantially free" from Y may be completely free from Y. Where necessary, the word "substantially" may be omitted from the definition of the invention.

As used herein, the term "each," when used in reference to a collection of items, is intended to identify an individual item in the collection but does not necessarily refer to every item in the collection. Exceptions can occur if explicit disclosure or context clearly dictates otherwise.

The use of any and all examples, or exemplary language (*e.g.,* "such as") provided herein, is intended merely to better illuminate the invention and does not pose a limitation on the scope of the invention unless otherwise claimed. No language in the specification should be construed as indicating any non-claimed element as essential to the practice of the invention.

All methods described herein are performed in any suitable order unless otherwise indicated herein or otherwise clearly contradicted by context. In regard to any of the methods provided, the steps of the method may occur simultaneously or sequentially. When the steps of the method occur sequentially, the steps may occur in any order, unless noted otherwise.

In cases in which a method comprises a combination of steps, each and every combination or sub-combination of the steps is encompassed within the scope of the disclosure, unless otherwise noted herein.

### IV. EXAMPLES

### EXAMPLE 1

This example describes the materials and methods to be used in the subsequent examples.

**Mice.** C57BL/6/J, *FoxP3*^{YFP-Ce}, Rag1^{-/-}(B6.129S7-Rag1^{tm1MoM}/J) mice were purchased from Jackson Laboratories. CD36fl/fl mice were generated as previously described (Son, N. H. et al. J Clin Invest 128, 4329-4342 (2018).). PPARγ^{fl/fl} and PPARβ^{fl/fl} mice were generated as described in Dammone, G. *et al.* (Dammone, G. et al. International journal of molecular sciences 19 (2018)). BRafCA; Tyr::CreER; Ptenlox4-5 (Braf/Pten) was described inDankort*etal.* (Dankort et al. Nature Genetics volume 41, pages544-552 (2009)). *K*-*ras*^{LSL-G12D/+}/*p53*^{fl/fl} conditional mouse model of NSCLC was described in DuPage *et al.* (DuPage et al. Nature Protocols volume 4, pages1064-1072 (2009)). Animals were housed in specific-pathogen-free facilities at the University of Lausanne, and all experimental studies were approved and performed in accordance with guidelines and regulations implemented by the Swiss Animal Welfare Ordinance.

**Cell lines and *in vitro* cultures.** YUMM1.7 melanoma cell line was described in Meeth, K., *et al.* (Meeth, K., et al. Pigment cell & melanoma research 29, 590-597, (2016)). YUMM1.7 and B16-ova melanomacell lines were cultured in DMEM with 10% fetal bovine serum and 1% penicillin-streptomycin and used for experiments when in exponential growth phase. MC38 colon adenocarcinoma cell line was described in Hoves *et al.* (Hoves et al. Journal of Experimental Medicine, 215 (3) 859-876 (2018)). The MC38 cell line was maintained in IMDM with 10% fetal bovine serum and 1% penicillin-streptomycin.

**Cancer cell-conditioned medium and iTreg culture.** iTregs were generated by activating naive CD4⁺ T cells with Dynabeads conjugated with anti-CD3 and anti-CD28 mAbs (ThermoFisher) in RMPI media supplemented with 10% FBS, 10ng/ml TGFβ and 50U/ml IL-2 for three days. Then activated CD4⁺ T cells were maintained in RPMI media plus 10% FBS and 50U/ml IL-2 for another 2 days. Differentiated iTregs were firstly sorted by using FACS cell sorter and then incubated in indicated culture condition for 48h. The survival and NAD/NADH levels of iTregs were then determined by live/dead staining and ELISA kits, respectively. For NAD/NADH measurement in CD36-KO iTregs treated with PPARβ agonist, sorted iTregs were cultured in cancer cell-conditioned medium in the presence of DMSO or GW50156 for 48h. Control RPMI for iTreg *in vitro* treatment was prepared with RPMI 1640 medium (Biological Industries) supplementing with 2mM Glucose, 10mM Glutamine, 10% Dialyzed FBS, 0.1% β-ME, and indicated levels of lactic acids. YUMM1.7 cancer cell-conditioned medium was collected by incubating YUMM1.7 cells (70-80% density) with control RPMI described above for 18h. Then, culture medium was collected and centrifuged at 2000 rpm for 15min to remove debris and cancer cells as cancer cell-conditioned medium. YUMM1.7 cancer cell-conditioned medium collected as described above was treated with Cleanascite^{™} reagent (Biotech Support Group) prior to Treg culture at a volume ratio of 1:5 according to the manufacturer's instruction.

***Ex vivo* suppression assay.** CD8 T cells from spleen of Ly5.1 mice were enriched using negative selection kit (MojoSort Mouse CD8 T cell isolation kit, Biolegend), and stained with CellTrace^{™} CFSE cell proliferation kit (ThermoFisher) for 15min at 37°C. 1 × 10⁴ CD8 cells were seeded into 96 well round plate in RPMI medium consisting of 50U/ml IL-2. CD44⁺/YFP⁺ Tregs (CD45.2⁺) isolated from splenocytes or TILs of *FoxP3*^{YFP-Cre} or Treg^{CD36-/-} mice were added according to indicated ratios for TregTeff. Then, anti-CD3/CD28-conjugated Dynabeads (ThermoFisher) were supplemented into cultures except for negative control groups. Cells were incubated at 37°C, 5% CO₂ for 72 h and then the proliferation of CD8⁺ T cells was determined by CFSE dilution with flow cytometry analysis.

**Tumor engraftment and treatment of tumor-bearing mice.** For tumor induction, 3-week-old Braf/Pten mice were treated with 4-hydroxytamoxifen on the skin surface as described before to induce tumor formation (Ho, P. C. et al. Cancer Res 74, 3205-3217 (2014)). For tumor engraftment, 5×10⁴ cells YUMM1.7, B16-OVA, or one million MC38 tumor cells were injected subcutaneously in 50µl PBS. Tumors were measured every 2-3 days post tumor engraftment or indicated treatments and calculated. Tumor volume was calculated by volume = (length × width²)/2 for engrafted tumor or volume= (length × width × height) for inducible tumor. For *in vivo* treatment, Yumm1.7-bearing mice were administrated every 3 days with either DMSO or PPARβ agonist (GW 501516) (1 mg per kg of body weight, Cayman Chemical) by intraperitoneal injection. For antibody-based treatment, tumor-bearing mice were treated with anti-PD-1 antibody (200 µg per injection, BioXcell, clone 29F.1A12) and anti-CD36 antibody (200 µg per injection, clone CRF D-2712 (Driscoll, W. S., et al. Circulation research 113, 52-61 (2013)) according to indicated combination by intraperitoneal injection. For antibody treatment in the Braf/Pten mouse model, four weeks after tumor induction, tumor-bearing Braf/Pten mice were treated with anti-CD36 antibody and/or anti-PD-1 antibody as indicated above for a period of 10 days. All experiments were conducted according to Swiss federal regulations.

**Tumor digestion and cell isolation.** Tumors were minced into small pieces in RPMI containing 2% FBS, 1% penicillin-streptomycin (p/s), DNase I (1 µg/ml, Sigma-Aldrich), and collagenase (0.5 mg/ml, Sigma-Aldrich) and kept for digestion for 40 min at 37°C, followed by the filtration with a 70 µm cell strainer. Filtered cells were incubated with ACK lysis buffer (Invitrogen) to lyse red blood cells and then washed with fluorescent activated cell sorter (FACS) buffer (phosphate-buffered saline with 2% fetal bovine serum and 2 mM EDTA). Tumor-infiltrating leukocytes were further enriched by percoll density gradient centrifugation (800 x g, 30 min) at room temperature as described before (Cheng, W. C. et al. Nat Immunol 20, 206-217 (2019)).

**Flow cytometry, cell sorting, and antibodies.** Single cell suspensions were incubated with Fe receptor-blocking anti-CD16/32 (93) and anti-CD351 (TX61) antibodies (Biolegend) on ice for 10 min before staining. Cell suspensions were first stained with LIVE/DEAD^{®} Fixable Violet Dead Cell Stain Kit (ThermoFisher) at 37°C for 10 min. After washing, surface proteins were stained for 30 min at 4°C. To detect cytokine production upon *ex vivo* re-stimulation, cell suspensions were re-suspended in RPMI 1640 with 10% FBS and then added to plates coated with 1 µg/ml anti-CD3 antibody (clone 145-2C11, Biolegend) and anti-CD28 antibody (clone 37.51, Biolegend) for another 5h at 37°C in the presence 2.5 µg/ml Brefeldin A Solution (BFA) (Biolegend). Cells were processed for surface marker staining as described above and then intracellular cytokine staining. Samples were analyzed on LSRII flow cytometers (BD Biosciences) and data were analyzed with FlowJo. Cells were sorted either on FACSAria^{™} III sorter (BD Biosciences) or SH800S Cell Sorter (Sony). The following antibodies against mouse proteins were used: anti-CD45 (30-F11), anti-CD3ε (17A2), anti-CD4 (RM4-5), anti-CD8a (53.6.7), anti-CD44 (IM7), anti-62L (Mel-14), anti-PD1 (RMP1-30), anti-CD134 (OX40) (OX-86), anti-CD357 (GITR) (DTA-1), anti-CD36 (CRF D-2712), anti-IgA (mA-6E1), anti-FoxP3 (MF-14), anti-IFN-γ (XMG1.2), anti-TNF-α (MP6-XT22), anti-IL17A (TC11-18H10.1), anti-Ki67 (16A8), anti-CD278 (ICOS) (15F9), anti-CD152 (CTLA4) (UC10-4B9), cleaved Caspase-3 (Asp175). These antibodies were purchased from Biolegend, eBiosciences and Cell Signaling.

**Mitochondrion, fatty acid uptake, and lipid content assay.** For measuring mitochondrial membrane potential, cells were washed and incubated with pre-warmed (37°C) staining solution (RPMI with 2% FBS) containing MitoTracker^{®} Deep Red FM (ThermoFisher) and MitoTracker^{®} Green FM (ThermoFisher) at the working concentrations of 10nm and 100nM for 15min, respectively. After staining, the cells were washed and resuspended in fresh FACS buffer for surface marker staining as described above. For measuring fatty acid uptake, cells were incubated in RPMI medium (or human T cell culture medium) containing C1-BODIPY^{®} 500/510 C12 (Life Technologies) at final concentration of 0.5µM for 15 min at 37°C. After incubation, cells were washed with FACS buffer for surface staining. For lipid content detection, after permeabilization and fixation, cells were stained using BODIPY^{®} 493/503 (Life Technologies) at a final concentration of 500ng/ml together with other intracellular proteins.

**RNA sequencing and bioinformatics analysis.** 500-600 viable CD4⁺/CD44⁺/YFP⁺ intratumoral Tregs from *FoxP3*^{YFP-Cre} or Treg^{CD36-/-} mice were isolated by FACS cell sorters (with at least 99% purity) directly into 4 µl lysis buffer consisting of 0.2 % (vol/vol) Triton X-100 solution (MgBCH-Axon Lab) and RNase inhibitor (Clontech). Plates containing samples were sealed, flash-frozen and kept at -80 °C before further processing following a version of the Smart-Seq2 protocol described before (Picelli, S. et al. Nature protocols 9, 171-181 (2014)). The RNA-sequencing raw data were processed through the standard RNA-seq analysis pipeline. Briefly, read alignment was examined using tophat2 v2.1.0 and then compared to the Mus musculus GRCm3 8. p4 genome version. Following the alignment, reads mapped to each gene were annotated using HTseq count. The differential expression analyses were conducted based on the DESeq2 R library. The differential expression test and visualization were then examined by the START Web-based RNA-seq analysis resources (Nelson, J. W., et al. Bioinformatics 33, 447-449 (2017).). Gene Set Enrichment Analysis (GSEA) was performed using GSEA software.

**Electron microscopy analysis and histology analysis.** For the electron microscopy analysis, sorted cells were fixed in glutaraldehyde 2.5% (EMS) and osmium tetroxide 1% (EMS) overnight at 4°C, followed by several washes with water and acetone (Sigma) and embedded in Epon (Sigma) resin the following day. Before imaging, 50nm slides were prepared by using a Leica Ultracut microtome and were contrasted using uranyl acetate (Sigma) and Reynolds lead citrate (Sigma). Electron microscope images were taken with a transmission electron microscope Philips CM100 at an acceleration of 80kV with a TVIPS TemCam-F416 digital camera with a magnification of 4800x and 11'000x. Image analysis and quantification were carried out using EMMENU, 3dmod (University of Colorado) and Fifi (ImageJ) software. To quantify mitochondria per sorted cell, a grid was applied and each intersection was defined as being part of the nucleus, cytoplasm or mitochondria and the length of each crista was measured divided by the mitochondrial area for determination of the crista density. For histology analysis, organs were trimmed and placed in the labeled cassettes and fixed in formalin for 24h for further embedding in molten paraffin wax. Paraffin sections at a thickness of 3-5µm were stained with hematoxylin and eosin according to standard procedures. Images were taken and exported on a Nikon Eclipse Ti-S inverted microscope.

**Human patient assessment.** This study was performed in accordance with the guidelines of ethic regulation for human samples under approved protocols. Human samples were analyzed following safety regulation and stained with the following antibodies for FACS analysis: anti-CD45 (2D1), anti-CD3 (SK7), anti-CD4 (SK3), anti-CD25 (BC96), anti-CD8 (RPA-TP), anti-CD36 (TR9), anti-PD1 (E12.1), and anti-FoxP3 (150D).

**T cell transfer model of colitis.** WT and CD36-KO Tregs were sorted from the spleens of either *FoxP3*^{YFP-Cre} mice or Treg^{CD36-/-} mice and naive CD4⁺ T cells were harvested using a combination of negative magnetic selection (Mo joSort Mouse CD4 T cell isolation kit, Biolegend) and FACS sorting (>98% purity). To induce colitis, naive CD4⁺ cells (5 × 10⁵ cells) were transferred intravenously into Rag1^{-/-}recipients. In some recipients, 4 × 10⁵ CD44⁺/YFP⁺ Tregs isolated from splenocytes of *FoxP3*^{YFP-Cre} mice or Treg^{CD36-/-} mice were co-transferred with naive CD4⁺ T cells. Recipient mice were monitored and weighted every two or three days after transferring for signs of diseases such as weight loss. Diseases onset usually occurs at 4-5 weeks post-transfer. The endpoints of this study included the determination of body weight loss, colitis length, and diarrhea. In addition, colons and small intestines were collected and processed for further evaluation by Haemotoxylin and Eosin staining.

**NAD and NADH measurement.** The ratio of nicotinamide adenine dinucleotide (NAD) and nicotinamide adenine dinucleotide hydrate (NADH) was measured by using the commercial NAD/NADH Quantification Kit (Sigma-Aldrich MAK037). The cells were first deproteinized to prevent the NAD and NADH consumption by enzymes. After washing with cold PBS, cell pellets suspended in NADH/NAD extraction buffer (200µl) were treated with two repetitive freeze-thaw cycles and then spun at 13,000×g for 5min at 4°C. The supernatant was then divided into two aliquots, one was for NADₜₒₜₐₗ detection, and the other one was heated at 60° C for 30 min for NAD decomposition. The samples were then transferred to a 96 wells plate for measuring the absorbance of 450nm. The amount of oxidized NAD (NAD⁺) was presented subtracting NADH from NADₜₒₜₐₗ. The ratio of NAD/NADH in a sample could be determined by the following equation: ratio = (NADₜₒₜₐₗ - NADH)/NADH.

**Seahorse extracellular flux analyses.** Extracellular flux analysis was performed with an XF96 Seahorse Extracellular Flux Analyzer as described before (Liu, P. S. et al. Nat Immunol 18, 985-994 (2017)) with minor modifications. Cells were treated with oligomycin (0.5µM, Sigma-Aldrich), FCCP (2µM, Sigma-Aldrich), rotenone (0.5µM, Sigma-Aldrich), antimycin A (0.5µM, Sigma-Aldrich), Glucose (10mM, Sigma-Aldrich) and 2-DG (50mM, Sigma-Aldrich). Each condition was performed with 3-6 replicates in each single experiment.

**Statistical analyses.** Statistical analyses were performed using the two-tailed, unpaired, Student's t-test. Log-rank (Mantel-Cox) test was used for survival curve analysis. Each point represented a biological replicate, and all data were presented as the mean± SD, or mean± SEM as indicated. TheP values were represented as follows: ****P* < 0.001, ***P* < 0.01 and **P* < 0.05. *P* <0.05 was considered statistically significant.

**Data availability.** The RNA-seq data for intratumoral Tregs are available in the Gene Expression Omnibus database.

### EXAMPLE2

### Intratumoral Tregs increased lipid metabolism and CD36 expression

To elucidate whether intratumoral Tregs preferentially engage specific metabolic pathways, the RNA-sequencing results from intratumoral and circulating Tregs obtained from breast cancer patients in a previously published study was first analyzed (Plitas, G. et al. Immunity 45, 1122-1134). Gene pathway analysis, with a particular focus on metabolic pathways, revealed that intratumoral Tregs highly expressed metabolic genes responsible for lipid metabolism when compared to circulating Tregs (**Figs. 1a and 1b**), suggesting that intratumoral Tregs may increase their lipid metabolism. Indeed, the comparison between peripheral blood mononuclear cells (PBMCs) and intratumoral Tregs from non-small-cell lung carcinoma (NSCLC) patients shows that intratumoral Tregs internalized a higher amount of a green fluorescent fatty acid, Bodipy FL C12, and contained a higher neutral lipid content based on the staining of Bodipy. To further explore these phenotypes, melanoma cell engraftment models were used to assess lipid metabolism in Tregs residing in tumors and other peripheral tissues. The results showed that intratumoral Tregs displayed an elevated ability to take up fatty acids (**Fig. 1c**) and had a higher lipid content **(****Fig. 1c**) compared to Tregs from other tissues of YUMM1.7 melanoma-bearing mice. Similarly, intratumoral Tregs of B16 melanoma-bearing mice also exhibited enhanced fatty acid uptake. These observations suggest that the increase in lipid metabolism by intratumoral Tregs is a conserved phenotype in both human and murine models. Of note, among genes controlling lipid uptake, CD36, a scavenger receptor responsible for long-chain fatty acid and oxidized low-density lipoprotein uptake, was significantly up-regulated in intratumoral Tregs compared to circulating Tregs from breast cancer patients (Plitas, G. et al. Immunity 45, 1122-1134). By examining Tregs in PBMC and tumor-infiltrating lymph nodes (TILN) from melanoma patients, it was confirmed that intratumoral Tregs from the majority of patients expressed higher levels of CD36 (**Fig. 1c**). In addition, intratumoral Tregs, but not Tregs residing in other peripheral tissues or secondary lymphoid organs from Yumm1.7 melanoma-bearing mice, expressed high levels of CD36 **(****Fig. 1f**). Notably, the increased expression of CD36 found in intratumoral Tregs was also observed in a B16 melanoma model, a genetically engineered Braf/PTEN melanoma mouse model, and a *K-ras*^{LSL-G12D/+}/*p53*^{fl/fl} conditional mouse model of NSCLC. Furthermore, culturing inducible Tregs (iTregs) in conditioned medium obtained from cancer cell cultures drastically increased CD36 expression, while hypoxia and lactic acid failed to induce CD36 expression in Tregs. Of note, lipid removal abolished the effects of cancer cell-conditioned media on stimulating CD36 expression in Tregs. Together, the results suggest that the TME can stimulate CD36 expression in Tregs, which can support the demands for metabolic adaptation in intratumoral Tregs.

### EXAMPLE3

### CD36 controls the accumulation and suppressive function of intratumoral Tregs

To investigate whether the expression of CD36 modulates Treg behavior in tumors, Treg-specific CD36-deficient mice (designated Treg^{CD36-/-}) were generated by crossing *CD36*^{fl/fl} mice with *Foxp3*^{YFP-Cre} mice. Given that genetic ablation of critical regulators in Tregs could lead to systemic activation of T lymphocytes and autoimmunity due to impairment of Treg suppressive functions, whether deficiency of CD36 in Treg impacts immune homeostasis was first examined. It was found that aged Treg^{CD36-/-} mice (21-23 weeks) displayed comparable body weights to *Foxp3*^{YFP-Cre} mice (referred to wild type mice throughout this study) in both genders. Treg^{CD36-/-} mice also contained a similar proportion of effector or memory population (CD44^{hi}CD62L^{lo}) in both CD4⁺ and CD8⁺ T cell compartments compared to WT mice. Moreover, Treg^{CD36-/-} mice showed neither abnormal infiltration of lymphocytes and myeloid cells in various organs nor severe systemic inflammatory disorders **(****Fig. 2a****),** suggesting that CD36 is not required for Tregs to maintain immune homeostasis.

YUMM1.7 melanoma cells were then engrafted into WT and Treg^{CD36-/-} mice. It was observed that genetic ablation of CD36 in Tregs drastically decreased lipid uptake and content in intratumoral Tregs, but not splenic Tregs **(****Figs. 2b** and **2c****),** indicating that intratumoral Tregs rely on CD36 expression to support enhanced lipid uptake. Growth deceleration of engrafted YUMM1.7 melanoma (**Figs. 2d** and **2e**), B16 melanoma, and MC38 colon carcinoma in Treg^{CD36-/-} mice were also observed. Moreover, Treg^{CD36-/-} mice had a profound loss of intratumoral Tregs, but not Tregs in spleen and draining lymph nodes at the endpoint of analyses **(****Fig. 2f****).** This was accompanied by a significant increase in the frequency of CD8⁺ TILs as well as the ratio of CD8⁺ to Treg TIL, a favorable parameter associated with strong anti-tumor responses. In addition, a higher frequency of CD8⁺ TILs and CD4⁺/FoxP3⁻ TILs in Treg^{CD36-/-} mice produced anti-tumor effector cytokines, including interferon-γ (IFNγ and tumor necrosis factor-α (TNFα) (**Fig. 2g**), suggesting that the TME of Treg^{CD36-/-} mice is less immunosuppressive.

To further validate the dependency of CD36 on supporting accumulation of intratumoral Tregs, heterozygous *Foxp3*^{YFP*-*Cre/+}/*CD36*^{fl/fl} female mice were generated, which simultaneously harbor a WT Treg population and a CD36-knockout Treg population driven by FoxP3 expression mediated by X chromosome inactivation. The WT and CD36-deficient Tregs can be detected on the basis of the expression of yellow fluorescent protein (YFP). To exclude the potential toxicity induced by Cre recombinase, heterozygous *Foxp3*^{YFP-Cre/+} female mice were also generated as control mice. By comparing YFP⁺ populations among FoxP3⁺ Tregs in both tumor-bearing *Foxp3*^{YFP-Cre/+}/*CD36*^{fl/fl} and *Foxp3*^{YFP-Cre/+} female mice, only a reduction in frequencies of CD36-deficient Tregs (Cre⁺ population in *Foxp3*^{YFP-Cre/-}/*CD36*^{fl/fl} mice) in tumors was detected, whereas the frequency ratios between Cre⁺ and Cre⁻ Tregs in both spleens and draining lymph nodes were comparable between *Foxp3*^{YFP-Cre/+}/*CD36*^{fl/fl} and *Foxp3*^{YFP-Cre/+} mice. This result suggested that loss of CD36 expression selectively perturb accumulation of intratumoral Tregs via intrinsic regulations. Altogether, these results reveal a crucial role for CD36 in selectively endowing Tregs with the ability to accumulate in the TME.

### EXAMPLE4

### CD36 was dispensable in Tregs for maintaining periphery homeostasis

Intriguingly, it was also found that intratumoral effector Tregs (CD44^{hi}/CD62L^{lo}) expressed higher levels of CD36 compared to intratumoral CD44^{lo} Tregs (resting Tregs) in murine melanoma model. Similarly, a higher percentage of tumor-infiltrating GITR⁺/CD25⁺ effector Tregs, the most suppressive subset of effector Tregs, from TILs of melanoma patients expressed CD36 compared to GITR⁺/CD25⁺ effector Tregs in PBMCs from melanoma patients and healthy donors. The expression of immunomodulatory receptors in intratumoral Tregs was also examined. It was found that CD36-KO Tregs reduced the expression of glucocorticoid-induced TNFR-related protein (GITR) and OX40, but not programmed cell death protein 1 (PD-1), CD25, cytotoxic T-lymphocyte-associated protein 4 (CTLA-4), or inducible T-cell costimulator (ICOS), compared to WT Tregs. These results suggest that CD36 expression contributes to the suppressive functions of effector Tregs. In support of this notion, intratumoral Tregs from Treg^{CD36-/-} mice displayed reduced suppressive capacity compared to WT intratumoral Tregs in the *ex vivo* suppressive assay **(****Fig. 2h****).** However, WT and CD36-deficient splenic Tregs displayed comparable suppressive capacity **(****Fig. 2i****),** suggesting that CD36 is only required for supporting the suppressive activity of intratumoral Tregs, but not splenic Tregs.

To further examine if CD36 is dispensable for Tregs to restraint peripheral inflammation, the ability of CD36-deficient Tregs on suppressing T cell transfer-induced colitis was analyzed. Disease onset such as weight loss was detected at two weeks post naive CD4⁺ T cell transfer. However, co-transferring CD36-KO Tregs was able to ameliorate weight loss in the recipient mice comparable to WT Tregs **(****Fig. 2j****).** Moreover, several organs were removed at the study endpoint and processed for further evaluation by histology. Compared with naive CD4⁺ T cell transfer, which triggered massive infiltration of lymphocytes and myeloid cells, co-transfer of either WT or CD36-KO Tregs impeded infiltration of lymphocytes and myeloid cells as well as morphology changes in colon and small intestine, colon shortening, and enlargement of spleen in the recipient mice. Furthermore, it was found that genetic ablation of CD36 neither affected the expression of activation markers, including CD44, CD103, and KLRG1, nor FoxP3 expression (indicated by the fluorescence intensity of YFP) in intratumoral Tregs. However, CD36-deficient intratumoral Tregs slightly enhanced the production of pro-inflammatory cytokines IFNγ and TNF, suggesting that CD36 restraints the ability to produce pro-inflammatory cytokine in intratumoral Tregs. Together, the results suggest that CD36 expression specifically supports intratumoral Tregs suppressive functions.

### EXAMPLE 5

### CD36 deficiency stimulated apoptosis in intratumoral Tregs

To investigate the underlying basis for the reduced cellularity of CD36-deficient Tregs in the TME, the proliferative ability was first examined by staining Ki67. It was found that CD36 deficiency did not alter proliferation in intratumoral Tregs. The comparison between the transcriptomes of WT and CD36-deficient intratumoral Tregs showed that CD36-deficient Tregs displayed elevated expression of genes controlling apoptosis **(****Fig. 3a****).** Indeed, higher levels of cleaved caspase-3 **(****Fig. 3b****)** and Annexin V staining **(****Fig. 4h****)** in CD36-deficient intratumoral Tregs were observed. Notably, CD36 deficiency did not enhance cleaved caspase-3 levels in Tregs from thymus and other secondary lymphoid organs, except a slight increase in draining lymph nodes, indicating that intratumoral Tregs require CD36-mediated regulation to prevent apoptosis.

Since mitochondrial metabolism and fitness have been suggested to modulate Treg suppressive function and survival (Yang, K. et al. Nature 548, 602-606 (2017); Weinberg, S. E. et al. Nature 565, 495-499 (2019); He, N. et al. PNAS 114, 12542-12547 (2017); Beier, U. H. et al. FASEB J 29, 2315-2326 (2015)), whether CD36-deficient intratumoral Tregs fail to sustain mitochondrial fitness was then examined. Strikingly, compared to WT Tregs, CD36-deficient intratumoral Tregs, but not Tregs in other tissues, displayed reduced mitochondrial membrane potential as measured by MitoTracker DeepRed staining **(****Fig. 3c****).** This observation was further supported by electron microscopy analysis which shows intratumoral Tregs from Treg^{CD36-/-} mice had fewer mitochondria **(****Fig. 3d****)** as well as fewer cristae in each mitochondrion **(****Fig. 3e****).** Nevertheless, WT and CD36-deficient splenic Tregs displayed comparable numbers of mitochondria and crista density. To further elucidate the impacts of CD36 on Treg mitochondrial metabolism, iTregs generated from either WT or CD36-deficient CD4⁺ T cells were treated with cancer cell-conditioned medium to induce CD36 expression as shown before. The Seahorse extracellular flux assays were performed. As shown in **Fig. 3f****,** CD36-deficient Tregs demonstrated reduced oxygen consumption rates (OCR) but increased glycolytic rates. These results suggest that CD36 ablation can impair oxidative phosphorylation (OXPHOS) and skew the metabolic preference of Tregs toward aerobic glycolysis. These observations suggested that enhanced CD36 expression in intratumoral Tregs may support Treg metabolic flexibility via modulation of mitochondrial fitness in response to the TME imposed metabolic stress (Li, X. et al. Nat Rev Clin Oncol (2019); Ho, P. C. et al. Cell 162, 1217-1228 (2015); Siska, P. J. & Rathmell, J. C. Trends Immunol 36, 257-264 (2015)).

Whether CD36-deficient Tregs displayed survival defects in response to metabolic challenges was also examined. In contrast to normal culture conditions (RMPI plus 10% FBS; designated RMPI), CD36-deficient Tregs exposed to cancer cell-conditioned medium exhibited reduced viability. Since lactic acid levels can be exacerbated in cancer cell-conditioned medium and the accumulation of lactic acid is a common feature of the TME, it was postulated that CD36-deficient Tregs might fail to sustain survival in this condition due to a high abundance of lactic acid. In support of this postulate, CD36-deficient Tregs was found to display a profound survival defect in response to escalating doses of lactic acids **(****Fig. 3g****).** In consistence with this result, a recent study suggested that elevated electron transport chain activity results in an increased NAD/NADH ratio to support lactic acid conversion into pyruvate in Tregs (Angelin, A. et al. Cell Metab 25, 1282-1293 e1287 (2017)), which can support survival of Tregs in a lactic acid-enriched condition. Thus, it was hypothesized that CD36-deficient Tregs may have a lower NAD/NADH ratio compared to WT Tregs due to reduced mitochondrial fitness and OXPHOS. Indeed, as shown in **Fig. 3h****,** CD36-deficient Tregs had a lower NAD/NADH ratio compared to WT Tregs, and supplementation with nicotinamide riboside (NR) to replenish NAD partially rescued the viability of CD36-deficient Tregs exposed to cancer cell-conditioned medium **(****Fig. 3i****).** Hence, the failure of CD36-deficient intratumoral Tregs to persist *in vivo* might result from reduced mitochondrial fitness and OXPHOS, which allow Tregs to survive in lactic acid-enriched conditions via a NAD-regulated metabolic process.

### EXAMPLE6

### CD36-PPARβ signaling orchestrated metabolic adaptation in intratumoral Tregs

To understand how CD36 stimulates mitochondrial fitness in intratumoral Tregs, changes in the transcriptomes of intratumoral and circulating Tregs from breast cancer patients were assessed. As expected, intratumoral Tregs up-regulated genes controlling mitochondrial functions and biogenesis. Moreover, intratumoral Tregs were found to display increased expression of genes involved in the PPAR signaling pathway **(****Fig. 4a****).** Since CD36 has been suggested to support metabolic flexibility in metabolic tissues by boosting PPARβ- (PPARβ also referred to as PPARβ) and PPARγ-dependent regulation of mitochondrial activity and biogenesis, CD36-induced metabolic reprogramming might promote mitochondrial fitness in intratumoral Tregs by providing lipid signals to adjust PPAR transcriptional regulation. To test this notion, both *PPAR*β^{fl/fl} and *PPARγ*^{fl/fl} mice with *Foxp3*^{YFP-Cre} mice were crossed to obtain Treg-specific PPARβ-deficient mice (designated Treg^{PPARβ-/-}) and PPARγ-deficient mice (designated Treg^{PPARγ-/-}), respectively. It was observed that genetic ablation of PPARγ in Tregs impaired neither accumulation of intratumoral Tregs nor YUMM1.7 melanoma growth. In contrast, Treg^{PPARβ-/-} mice recapitulated the characteristic features of Treg^{CD36-/-} mice, including reduced intratumoral Treg accumulation **(****Fig. 4b****),** growth deceleration of engrafted YUMM1.7 melanoma (**Figs. 4c** and **4d**), and an increase in CD8⁺ TILs. Similar to CD36-deficient intratumoral Tregs, PPARβ-deficient intratumoral Tregs displayed reduced mitochondrial membrane potential compared to WT intratumoral Tregs **(****Fig. 4e****).** Of note, PPARβ-deficient intratumoral Tregs expressed less CD36 compared to WT intratumoral Tregs. Given that lipid removal abolished the ability of cancer cell-conditioned media to induce CD36 expression in Tregs, the results further suggest that lipid-induced PPARβ signals might contribute to CD36 induction in intratumoral Tregs.

To elucidate the relationship between CD36 and PPARβ activation and their roles in supporting accumulation of intratumoral Tregs, YUMM1.7 melanoma-engrafted WT mice and Treg^{CD36-/-} mice were treated with either PPARβ selective agonist (GW501516) or control vehicle for two weeks. As shown in **Figs. 4f** and **4g****,** treatment with GW501516 restored tumor growth as well as intratumoral Treg abundance in Treg^{CD36-/-} mice **(****Figs. 4f** and **4g****).** In addition, intratumoral Tregs from GW501516-treated Treg^{CD36-/-} mice had increased mitochondrial membrane potentials and lower levels of cleaved caspase-3 **(****Figs. 4h** and **4i****).** In parallel, GW501516 treatment in CD36-deficient Tregs increased NAD/NADH ratios, indicating that CD36-controlled lipid uptake activates PPARβ pathways to support mitochondrial fitness and enhancement of NAD/NADH ratios in intratumoral Tregs. Moreover, the activation of PPARβ pathways may further amplify CD36-mediated metabolic adaptation in intratumoral Tregs by enhancing CD36 expression. Altogether, these results reveal that CD36-PPARβ signaling orchestrated metabolic programs to support Treg persistence in the TME.

### EXAMPLE 7

### CD36-targeting reinforced antitumor immunity by impairing intratumoral Tregs

Next, whether blocking CD36-mediated metabolic adaptation could specifically disturb intratumoral Tregs without systemic loss of Tregs and global impairment of Treg suppressive functions was investigated. Yumm1.7 melanoma-engrafted mice were treated with an anti-CD36 monoclonal antibody (mAb), which interferes with CD36-mediated fatty acid and oxidized low-density lipoprotein uptake. As shown in **Fig. 5a****,** anti-CD36 mAb treatment reduced tumor growth and was accompanied by reduced accumulation of intratumoral Tregs, while the Treg population was sustained in spleen and draining lymph nodes **(****Figs. 5b** and **6a****).** Similar to genetic ablation of CD36 in Tregs, anti-CD36 mAb treatment promoted apoptosis in intratumoral Tregs **(****Fig. 5c****)** and led to a significant increase in tumor infiltration of CD8⁺ T cells **(****Fig. 6b****).** In addition, treating mice with an anti-CD36 mAb improved the production of anti-tumor effector cytokines in CD8⁺ and CD4⁺ TILs **(****Figs. 6c** and **6d****).** Since CD36 expression can support metabolic flexibility and metastasis in cancer cells as well as other immune cells, the anti-tumor responses induced by anti-CD36 mAb may be Treg-independent. To test this notion, the same treatment using Treg^{CD36-/-} mice as recipients was performed, the results showed that anti-CD36 mAb treatment was incapable of suppressing tumor progression in Treg^{CD36-/-} mice **(****Fig. 5d****),** indicating that the anti-tumor responses induced by anti-CD36 mAb treatment might mainly result from targeting CD36 expressed in Tregs instead of other CD36-expressing cells.

As T cell exhaustion may limit the therapeutic outcomes of Treg-targeting interventions, it is possible that reinvigorating exhausted T cells with PD-1 blockade may potentiate the anti-tumor effects of CD36 blockade to restrain tumor progression. Indeed, anti-PD-1 mAb more effectively limited tumor progression and prolonged survival in tumor-bearing Treg^{CD36-/-} mice compared to WT mice **(****Figs. 5e** and **5f****).** In addition to genetic ablation of CD36 in Tregs, anti-PD-1 mAb also potentiated the anti-tumor responses of anti-CD36 mAb in both the genetically engineered Braf/PTEN melanoma mouse model **(****Fig. 5g****)** and the YUMM1. 7 engraftment model **(****Fig. 5h****).** These results demonstrate that targeting CD36 in Tregs might reprogram the TME towards more immunostimulatory conditions, which may therapeutically complement the effect of PD-1 blockade to counteract T-cell exhaustion. This points to CD36 blockade as a new potential immunotherapeutic intervention with reduced side effects caused by systemic impairment of Tregs.

The synergistic effect of the checkpoint blockade inhibitors (e.g., the PD1 and CTLA4 inhibitors) is further demonstrated in **Figs. 7a****-f.** For tumor engraftment, 5×10⁴ cells YUMM1.7 tumor cells were injected subcutaneously in 50µl PBS. Tumors were measured every 2~3 days post tumor engraftment or indicated treatments and calculated. Tumor volume was calculated by volume= (length × width²)/2. For antibody-based treatment, tumor-bearing mice were treated with anti-PD-1 antibody (200 µg per injection, BioXcell, clone 29F.1A12), anti-CTLA4 antibody (200 µg per injection, BioXcell, clone 9D9), and anti-CD36 antibody (200 µg per injection, clone CRF D-2712) according to indicated combination by intraperitoneal injection.

As shown in **Figs. 7a****,** **7c****,** **7d****,** and **7e****,** Yumm1.7 melanoma-engrafted mice were treated with anti-CD36 monoclonal antibody (mAb) and anti-CTLA4 mAb. It was found that anti-CD36 mAb treatment decreased tumor growth and was accompanied by reduced frequency of intratumoral Tregs, while the Treg population was sustained in spleen and draining lymph nodes, which was not able to achieve by the treatment of anti-CTLA4 mAb. Additionally, similar to the results shown in **Fig. 5h****,** anti-PD-1 mAb also potentiated the anti-tumor responses of anti-CD36 mAb in the YUMM1.7 engraftment model, though the combined treatment of anti-PD-1 mAb and anti-CTLA4 mAb also reduced the tumor growth. These results indicate that targeting CD36 in Tregs might reprogram the TME towards more immunostimulatory conditions, which may therapeutically counterpart the effect of PD-1 blockade to counteract tumor progression.

The results presented in this disclosure show that intratumoral Tregs upregulate expression of CD36 to facilitate fatty acid uptake. The internalized fatty acids further support mitochondrial fitness by activating PPARβ-mediated transcriptional programs that control mitochondrial biogenesis and functions. The enhanced mitochondrial fitness in CD36-expressing intratumoral Tregs leads to regeneration of NAD via electron transport chain complex I, which in turn sustain lactate → pyruvate conversion. As a result of continuous support of lactate → pyruvate conversion via NAD regeneration, intratumoral Tregs can survive in the acidic tumor microenvironment and may utilize lactate-derived pyruvate for supporting immunosuppressive activity.

Exploiting the regulatory circuits by which metabolic processes orchestrate immune responses in immune cells is an attractive strategy for fine-tuning host immunity in diseases. This disclosure demonstrated that CD36-PPARβ signaling sustains survival and functional fitness in intratumoral Tregs by modulating mitochondrial fitness and NAD levels. Owing to the uniqueness of the metabolic stress occurring in the TME and the selectivity of CD36-PPARβ signaling for intratumoral Tregs, targeting CD36 may provide broad therapeutic potential with a limited negative impact on immune and peripheral tissue homeostasis in cancer patients. Moreover, the additive anti-tumor effects elicited by combined treatment with PD-1 blockade and CD36 targeting further warrant the development of CD36 inhibition approaches as potential cancer treatments.

## Claims

1. A CD-36 inhibitor for use in a method of treating cancer by reducing the number of intratumoral CD4+ T regulatory cells, wherein the treatment inhibits tumor growth in a subject having cancer; and wherein the CD-36 inhibitor is an anti-CD36 antibody.

2. The CD-36 inhibitor for the use according to claim 1, wherein the antibody is to be administered intratumorally, intravenously, subcutaneously, intraosseously, in sustained release, in controlled release or in delayed release.

3. The CD-36 inhibitor for the use according to claim 1 or claim 2, wherein the cancer is selected from oral cancer, oropharyngeal cancer, nasopharyngeal cancer, respiratory cancer, urogenital cancer, gastrointestinal cancer, central or peripheral nervous system tissue cancer, an endocrine or neuroendocrine cancer or hematopoietic cancer, glioma, sarcoma, carcinoma, lymphoma, melanoma, fibroma, meningioma, brain cancer, renal cancer, biliary cancer, pheochromocytoma, pancreatic islet cell cancer, Li-Fraumeni tumors, thyroid cancer, parathyroid cancer, pituitary tumors, adrenal gland tumors, osteogenic sarcoma tumors, multiple neuroendocrine type I and type II tumors, breast cancer, lung cancer, head and neck cancer, prostate cancer, esophageal cancer, tracheal cancer, liver cancer, bladder cancer, stomach cancer, pancreatic cancer, ovarian cancer, uterine cancer, cervical cancer, testicular cancer, colon cancer, rectal cancer, and skin cancer.

4. The CD-36 inhibitor for the use according to any of claims 1 to 3, wherein the treatment further comprises the administration of an additional therapeutic agent.

5. The CD-36 inhibitor for the use according to claim 4, wherein the additional therapeutic agent comprises an immune checkpoint modulator.

6. The CD-36 inhibitor for the use according to claim 5, wherein the immune checkpoint modulator comprises an antibody specific for CTLA-4, PD-1, PD-L1, PD-L2, killer immunoglobulin receptor (KIR), LAG3, B7-H3, B7-H4, TIM3, A2aR, CD40L, CD27, OX40, 4-IBB, TCR, BTLA, ICOS, CD28, CD80, CD86, ICOS-L, HVEM, 4-1BBL, OX40L, CD70, CD40, or GALS.

7. The CD-36 inhibitor for the use according to claim 5 or claim 6, wherein the immune checkpoint modulator comprises an anti-PD-1 monoclonal antibody, anti-CTLA4 monoclonal antibody, or a combination thereof.

## Patentansprüche

1. CD-36-Inhibitor zur Verwendung in einem Verfahren zum Behandeln von Krebs durch Reduzieren der Anzahl intratumoraler regulatorischer CD4+-T-Zellen, wobei die Behandlung Tumorwachstum in einem Subjekt, das Krebs aufweist, hemmt; und wobei der CD-36-Inhibitor ein Anti-CD36-Antikörper ist.

2. CD-36-Inhibitor zur Verwendung nach Anspruch 1, wobei der Antikörper intratumoral, intravenös, subkutan, intraossär, in verzögerter Freisetzung, in kontrollierter Freisetzung oder in zeitverzögerter Freisetzung zu verabreichen ist.

3. CD-36-Inhibitor zur Verwendung nach Anspruch 1 oder Anspruch 2, wobei der Krebs ausgewählt ist aus Mundkrebs, Oropharyngealkrebs, Nasopharyngealkrebs, Atemwegskrebs, Urogenitalkrebs, gastrointestinalem Krebs, Krebs des zentralen oder peripheren Nervensystemgewebes, einem endokrinen oder neuroendokrinen Krebs oder hämatopoetischem Krebs, Gliom, Sarkom, Karzinom, Lymphom, Melanom, Fibrom, Meningeom, Hirnkrebs, Nierenkrebs, Gallengangskrebs, Phäochromozytom, Inselzellkrebs der Bauchspeicheldrüse, Li-Fraumeni-Tumoren, Schilddrüsenkrebs, Nebenschilddrüsenkrebs, Hypophysentumoren, Nebennierentumoren, osteogenen Sarkom-Tumoren, multiplen neuroendokrinen Tumoren vom Typ I und Typ II, Brustkrebs, Lungenkrebs, Kopf- und Halskrebs, Prostatakrebs, Speiseröhrenkrebs, Trachealkrebs, Leberkrebs, Blasenkrebs, Magenkrebs, Bauchspeicheldrüsenkrebs, Eierstockkrebs, Gebärmutterkrebs, Gebärmutterhalskrebs, Hodenkrebs, Kolonkarzinom, Rektumkarzinom, und Hautkrebs.

4. CD-36-Inhibitor zur Verwendung nach einem der Ansprüche 1 bis 3, wobei die Behandlung ferner die Verabreichung eines zusätzlichen therapeutischen Mittels umfasst.

5. CD-36-Inhibitor zur Verwendung nach Anspruch 4, wobei das zusätzliche therapeutische Mittel einen Immun-Checkpoint-Modulator umfasst.

6. CD-36-Inhibitor zur Verwendung nach Anspruch 5, wobei der Immun-Checkpoint-Modulator einen Antikörper umfasst, der spezifisch ist für CTLA-4, PD-1, PD-L1, PD-L2, Killer-Immunglobulin-Rezeptor (KIR), LAG3, B7-H3, B7-H4, TIM3, A2aR, CD40L, CD27, OX40, 4-IBB, TCR, BTLA, ICOS, CD28, CD80, CD86, ICOS-L, HVEM, 4-1BBL, OX40L, CD70, CD40 oder GALS.

7. CD-36-Inhibitor zur Verwendung nach Anspruch 5 oder Anspruch 6, wobei der Immun-Checkpoint-Modulator einen monoklonalen Anti-PD-1-Antikörper, einen monoklonalen Anti-CTLA4-Antikörper oder eine Kombination davon umfasst.

## Revendications

1. Inhibiteur de CD-36 destiné à être utilisé dans un procédé de traitement d'un cancer par réduction du nombre de cellules T régulatrices CD4+ intratumorales, ledit traitement inhibant la croissance tumorale chez un sujet atteint d'un cancer ; et ledit inhibiteur de CD-36 étant un anticorps anti-CD36.

2. Inhibiteur de CD-36 destiné à être utilisé selon la revendication 1, ledit anticorps devant être administré par voie intratumorale, intraveineuse, sous-cutanée, intraosseuse, à libération prolongée, à libération contrôlée ou à libération retardée.

3. Inhibiteur de CD-36 destiné à être utilisé selon la revendication 1 ou la revendication 2, ledit cancer étant choisi parmi le cancer buccal, le cancer de l'oropharynx, le cancer du nasopharynx, le cancer des voies respiratoires, le cancer urogénital, le cancer gastro-intestinal, le cancer du tissus du système nerveux central ou périphérique, un cancer endocrinien ou neuroendocrinien ou un cancer hématopoïétique, le gliome, le sarcome, le carcinome, le lymphome, le mélanome, le fibrome, le méningiome, le cancer du cerveau, le cancer du rein, le cancer biliaire, le phéochromocytome, le cancer des cellules des îlots pancréatiques, les tumeurs de Li-Fraumeni, le cancer de la thyroïde, le cancer de la parathyroïde, les tumeurs de l'hypophyse, les tumeurs des glandes surrénales, les ostéosarcomes, plusieurs tumeurs neuroendocrines de type I et de type II, le cancer du sein, le cancer du poumon, le cancer de la tête et du cou, le cancer de la prostate, le cancer de l'œsophage, le cancer de la trachée, le cancer du foie, le cancer de la vessie, le cancer de l'estomac, le cancer du pancréas, le cancer de l'ovaire, le cancer de l'utérus, le cancer du col de l'utérus, le cancer des testicules, le cancer du côlon, le cancer du rectum, et le cancer de la peau.

4. Inhibiteur de CD-36 destiné à être utilisé selon l'une quelconque des revendications 1 à 3, ledit traitement comprenant en outre l'administration d'un agent thérapeutique supplémentaire.

5. Inhibiteur de CD-36 destiné à être utilisé selon la revendication 4, dans lequel l'agent thérapeutique supplémentaire comprend un modulateur de point de contrôle immunitaire.

6. Inhibiteur de CD-36 destiné à être utilisé selon la revendication 5, dans lequel le modulateur de point de contrôle immunitaire comprend un anticorps spécifique de CTLA-4, PD-1, PD-L1, PD-L2, du récepteur de type immunoglobuline des cellules tueuses (KIR), LAG3, B7-H3, B7-H4, TIM3, A2aR, CD40L, CD27, OX40, 4-IBB, TCR, BTLA, ICOS, CD28, CD80, CD86, ICOS-L, HVEM, 4-1BBL, OX40L, CD70, CD40 ou GALS.

7. Inhibiteur de CD-36 destiné à être utilisé selon la revendication 5 ou la revendication 6, ledit modulateur de point de contrôle immunitaire comprenant un anticorps monoclonal anti-PD-1, un anticorps monoclonal anti-CTLA4 ou une combinaison de ceux-ci.
